# EUROPEAN PATENT APPLICATION

(11) **EP 4 424 318 A1**
(43) Date of publication of application: **04.09.2024**
(21) Application number: 22887626.4
(22) Date of filing: 26.10.2022
(51) Int. Cl.: A61K 35/74, A61P 17/00, A61P 17/06, A61P 17/10, A61P 29/00, A61P 31/04, A23L 33/135, A23K 10/16, C12N 1/20

(54) **COMPOSITION FOR PREVENTING, ALLEVIATING OR TREATING INFLAMMATORY SKIN DISEASES, COMPRISING CUTIBACTERIUM SP. STRAIN**

(30) Priority: 27.10.2021 KR 20210144908
(71) Applicant: CJ Bioscience, Inc., Jung-gu Seoul 04527 (KR)
(72) Inventor: SHIN, Changsik, Seoul 04560 (KR); LEE, Ji Young, Seoul 04560 (KR); KWON, Bo Eun, Seoul 04560 (KR); KIM, Hyun Jeong, Seoul 04560 (KR); KIM, Hanseol, Seoul 04560 (KR); PARK, Hyunkyung, Seoul 04560 (KR); CHOI, Joung Eun, Seoul 04560 (KR)
(74) Representative: Reddie & Grose LLP
(86) International application number: PCT/KR2022/016508
(87) International publication number: WO 2023/075430

(57) **Abstract**

The present application relates to a use of a Cutibacterium sp. strain, a culture product of the strain or a combination thereof for preventing, alleviating and/or treating inflammatory skin disease. A composition comprising the Cutibacterium sp. strain, the culture product of the strain or the combination thereof is non-cytotoxic, inhibits the proliferation of immune cells that induce skin inflammation, inhibits an immune response, has anti-bacterial effects on acne-causing bacteria, and promotes the regeneration of skin cells, and thus has the excellent effects of preventing, alleviating and/or treating inflammatory skin disease.

## Description

### [TECHNICAL FIELD]

The present application relates to a use of a *Cutibacterium* sp. strain, a culture product of the strain or a combination thereof for preventing, alleviating and/or treating inflammatory skin disease.

### [BACKGROUND ART]

Skin plays a protective barrier role which protects the human body from various kinds of oxidizing substances, air pollution, heavy metals, ultraviolet radiation (UV) and the like, prevents bacteria, fungi, viruses and the like from invading the skin, and prevents moisture loss. The structure of the skin is largely divided into 3 layers of epidermis, dermis and subcutaneous fat, and among them, the dermis layer accounting for 90% is composed of collagen, elastin, hyaluronic acid and glycoprotein. A major component, collagen is an important fibrous protein which accounts for approximately 25% of the total mass of the proteins of the human body, and accounts for most of connective tissue, and plays important functions of an intracellular matrix role, a structural support function, induction of division and differentiation of cells, provision of skin tensile strength and the like.

In addition, it is a well-known fact that fatty acids, higher alcohols, proteins and the like in the sebum components and sweat components which are excreted from the body and cosmetic components are decomposed into highly toxic substances by skin pathogens on the skin, and skin inflammation may be caused by them, and secondary infection exposure due to pathogenic microorganisms caused by skin barrier damage by ultraviolet rays of the sun causes various kinds of skin disease such as acne, itchiness, dermatitis, allergy outbreaks and the like.

In order to protect skin from such various harmful environments, discovery of a substance which effectively inhibits the growth of skin harmful bacteria and has no side effects and development of medicines and/or cosmetics applying thereof are needed.

### [PRIOR ART]

### [PATENT DOCUMENT]

(Patent document 1) KR 10-2032546 B1

### [DISCLOSURE]

### [TECHNICAL PROBLEM]

An object of the present application is to provide a *Cutibacterium* sp. strain comprising (a) an Lrp/AsnC family transcriptional regulator or a gene encoding the same, and
(b) a lactococcin 972 family bacteriocin or a gene encoding the same.

Another object of the present application is to provide a pharmaceutical composition for preventing or treating inflammatory skin disease, comprising the strain, a culture product of the strain, or a combination thereof.

Other object of the present application is to provide an antibacterial composition against inflammatory skin disease-causing bacterum, comprising the strain, a culture product of the strain, or a combination thereof.

Other object of the present application is to provide a food composition for preventing or alleviating inflammatory skin disease, comprising the strain, a culture product of the strain, or a combination thereof.

Other object of the present application is to provide a feed composition for preventing or alleviating inflammatory skin disease, comprising the strain, a culture product of the strain, or a combination thereof.

### [TECHNICAL SOLUTION]

The present applicant has diligently tried to develop a composition having effects on preventing, alleviating and/or treating inflammatory skin disease such as acne, rosacea, and the like, and as a result, it has been confirmed that a *Cutibacterium* sp. strain, a culture product of the strain, or a combination thereof has effects on preventing, alleviating and/or treating inflammatory skin disease, and thereby, the present application has been completed.

Accordingly, the present application provides a novel *Cutibacterium* sp. strain.

The present application provides a use of a *Cutibacterium* sp. strain, a culture product of the strain, or a combination thereof for using in preventing, alleviating and/or treating inflammatory skin disease.

One embodiment of the present application provides a composition for preventing, alleviating and/or treating inflammatory skin disease comprising a *Cutibacterium* sp. strain, a culture product of the strain, or a combination thereof. In one embodiment, the composition may be a pharmaceutical composition, a cosmetic composition, a food composition, or a feed composition.

Another embodiment provides a pharmaceutical composition for preventing, alleviating and/or treating inflammatory skin disease comprising a *Cutibacterium* sp. strain, a culture product of the strain, or a combination thereof.

Other embodiment provides a food composition for preventing and/or alleviating inflammatory skin disease comprising a *Cutibacterium* sp. strain, a culture product of the strain, or a combination thereof. The food composition may be a food additive or a health functional food.

Other embodiment provides a feed composition for preventing and/or alleviating inflammatory skin disease comprising a *Cutibacterium* sp. strain, a culture product of the strain, or a combination thereof. The feed composition may be a feed additive.

Other embodiment provides a cosmetic composition for preventing and/or alleviating inflammatory skin disease, or alleviating skin, comprising a *Cutibacterium* sp. strain, a culture product of the strain, or a combination thereof.

Other embodiment provides a method for preventing, alleviating and/or treating inflammatory skin disease comprising administering an effective dose of a *Cutibacterium* sp. strain, a culture product of the strain, or a combination thereof into a subject in need of preventing, alleviating and/or treating inflammatory skin disease.

Other embodiment provides a use of a *Cutibacterium* sp. strain, a culture product of the strain, or a combination thereof for using in preparation of a composition (for example, pharmaceutical composition, cosmetic composition, food composition, or feed composition) for preventing, alleviating and/or treating inflammatory skin disease.

Other embodiment provides a use of a *Cutibacterium* sp. strain, a culture product of the strain, or a combination thereof for inhibiting inflammatory skin disease-causing bacterum.

Other embodiment provides an antibacterial composition against inflammatory skin disease-causing bacterum comprising a *Cutibacterium* sp. strain, a culture product of the strain, or a combination thereof.

Other embodiment provides a method for inhibition against inflammatory skin disease causative bacteria comprising applying an effective dose of a *Cutibacterium* sp. strain, a culture product of the strain, or a combination thereof to a subject in need of antibacterial treatment against inflammatory skin disease-causing bacterum.

Other embodiment provides a use of a *Cutibacterium* sp. strain, a culture product of the strain, or a combination thereof for using in preparation of an antibacterial composition against inflammatory skin disease-causing bacterum.

Other embodiment provides a disinfectant comprising a *Cutibacterium* sp. strain, a culture product of the strain, or a combination thereof as an active ingredient.

Other embodiment provides a use of a *Cutibacterium* sp. strain, a culture product of the strain, or a combination thereof for using in preparation of a disinfectant.

Other embodiment provides a method for disinfection, comprising applying the disinfectant to a subject in need of disinfection.

Other embodiment provides a detergent comprising a *Cutibacterium* sp. strain, a culture product of the strain, or a combination thereof as an active ingredient.

Other embodiment provides a use of a *Cutibacterium* sp. strain, a culture product of the strain, or a combination thereof for using in preparation of a detergent.

Other embodiment provides a method for cleaning, comprising applying the detergent to a subject in need of disinfection.

Hereinafter, the present application will be described in more detail.

### Cutibacterium sp. strains and culture products thereof

One aspect provides a *Cutibacterium* sp. strain.

In the present description, the *Cutibacterium* sp. strain may comprise all Cutibacterium sp. strains. The *Cutibacterium* sp. Strain may be at least one (1 kind, 2 kinds, or 3 kinds) selected from the group consisting of *Cutibacterium acnes (C. acnes), Cutibacterium avidum (C. avidum),* and *Cutibacterium granulosum (C. granulosum),* but not limited thereto.

In one embodiment, the *Cutibacterium* sp. strain may be a *C. acnes* subspecies *acnes* (Type I), a *C. acnes* subspecies *defendens* (Type II), or a *C. acnes* subspecies *elongatum* (Type III), but not limited thereto. In one embodiment, the Cutibacterium sp. strain may be a *C. acnes* subspecies *defendens* (Type II).

In one embodiment, in the *Cutibacterium* sp. strain, the ribotype may be RT1, RT2, RT3, RT4, RT5, RT6, RT7, RT8, RT9, or RT10, but not limited thereto. In one embodiment, the Cutibacterium sp. strain may be RT2.

In one embodiment, in the *Cutibacterium* sp. strain, the sequence type may be 69 or 153.

In one embodiment, in the *Cutibacterium* sp. strain, the CC (clonal complex) may be CC72 (Type II).

In one embodiment, the *Cutibacterium* sp. strain, may comprise at least one (1 kind or 2 kinds) selected from the group consisting of
(a) an Lrp/AsnC family transcriptional regulator (NCBI Reference Sequence: WP_002531510.1) and/or a gene encoding the same, and
(b) a lactococcin 972 family bacteriocin (NCBI Reference Sequence: WP_070651130.1) and/or a gene encoding the same, and/or
not comprise at least one (1 kind, 2 kinds, 3 kinds, 4 kinds, 5 kinds, or 6 kinds) selected from the group consisting of the following (1) to (6) and/or genes encoding the same:
(1) beta-glucuronidase (NCBI Reference Sequence: WP_002518535.1) or a gene encoding the same,
(2) 2-isopropylmalate synthase (NCBI Reference Sequence: WP_142263178.1) or a gene encoding the same,
(3) 3-isopropylmalate dehydratase (NCBI Reference Sequence: WP_002513330.1) or a gene encoding the same,
(4) type I-E CRISPR-associated protein Cse1/CasA (NCBI Reference Sequence: WP_002514606.1) or a gene encoding the same,
(5) type I-E CRISPR-associated protein Cas7/Cse4/CasC (NCBI Reference Sequence: WP_002514608.1) or a gene encoding the same, and
(6) CRISPR-associated helicase/endonuclease Cas3 (NCBI Reference Sequence: WP_002514605.1) or a gene encoding the same.

In one embodiment, the *Cutibacterium* sp. strain, may comprise at least one (1 kind or 2 kinds) selected from the group consisting of
(a) an Lrp/AsnC family transcriptional regulator (NCBI Reference Sequence: WP_002531510.1) and/or a gene encoding the same, and
(b) a lactococcin 972 family bacteriocin (NCBI Reference Sequence: WP_070651130.1) and/or a gene encoding the same, and
does not comprise at least one (1 kind, 2 kinds, 3 kinds, 4 kinds, 5 kinds, or 6 kinds) selected from the group consisting of the following (1) to (6):
(1) beta-glucuronidase (NCBI Reference Sequence: WP_002518535.1) or a gene encoding the same,
(2) 2-isopropylmalate synthase (NCBI Reference Sequence: WP_142263178.1) or a gene encoding the same,
(3) 3-isopropylmalate dehydratase (NCBI Reference Sequence: WP_002513330.1) or a gene encoding the same,
(4) type I-E CRISPR-associated protein Cse1/CasA (NCBI Reference Sequence: WP_002514606.1) or a gene encoding the same,
(5) type I-E CRISPR-associated protein Cas7/Cse4/CasC (NCBI Reference Sequence: WP_002514608.1) or a gene encoding the same, and
(6) CRISPR-associated helicase/endonuclease Cas3 (NCBI Reference Sequence: WP_002514605.1) or a gene encoding the same.

In one embodiment, the *Cutibacterium* sp. strain, may comprise
(a) an Lrp/AsnC family transcriptional regulator (NCBI Reference Sequence: WP_002531510.1) or a gene encoding the same, and
(b) a lactococcin 972 family bacteriocin (NCBI Reference Sequence: WP_070651130.1) or a gene encoding the same, and
does not comprise all of proteins of the following (1) to (6) or genes encoding the same:
(1) beta-glucuronidase (NCBI Reference Sequence: WP_002518535.1) or a gene encoding the same,
(2) 2-isopropylmalate synthase (NCBI Reference Sequence: WP_142263178.1) or a gene encoding the same,
(3) 3-isopropylmalate dehydratase (NCBI Reference Sequence: WP_002513330.1) or a gene encoding the same,
(4) type I-E CRISPR-associated protein Cse1/CasA (NCBI Reference Sequence: WP_002514606.1) or a gene encoding the same,
(5) type I-E CRISPR-associated protein Cas7/Cse4/CasC (NCBI Reference Sequence: WP_002514608.1) or a gene encoding the same, and
(6) CRISPR-associated helicase/endonuclease Cas3 (NCBI Reference Sequence: WP_002514605.1) or a gene encoding the same.

In one embodiment, the *Cutibacterium* sp. strain may comprise a gene consisting of SEQ ID NO: 8 as 16S rRNA.

In one embodiment, the *Cutibacterium* sp. strain may be selected from the group consisting of the followings:
CJRS-1 0651,
CJRS-10652,
CJRS-10653,
CJRS-10654,
CJRS-10655, and
CJRS-10656.

The strain selected from the group consisting of the CJRS-10651 strain, CJRS-10652 strain, CJRS-10653 strain, CJRS-10654 strain, CJRS-10655 strain, and CJRS-10656 strain, may comprise 16S rRNA of SEQ ID NO: 8 in common.

The CJRS-10651 strain may be Accession number KCCM13032P (named *"Cutibacterium acnes* CJRS-10651" by a depositor).

The CJRS-10652 strain may be Accession number KCCM13033P (named *"Cutibacterium acnes* CJRS-10652" by a depositor).

The CJRS-10653 strain may be Accession number KCCM13034P (named *"Cutibacterium acnes* CJRS-10653" by a depositor).

The CJRS-10654 strain may be Accession number KCCM13035P (named *"Cutibacterium acnes* CJRS-10654" by a depositor).

The CJRS-10655 strain may be Accession number KCCM13036P (named *"Cutibacterium acnes* CJRS-10655" by a depositor).

The CJRS-10656 strain may be Accession number KCCM13037P (named *"Cutibacterium acnes* CJRS-10656" by a depositor).

In the present description, "culture product" of a *Cutibacterium* sp. strain may mean a product obtained by culturing a *Cutibacterium* sp. strain. The culture product of the *Cutibacterium* sp. strain may be in a form in which the strain (microbial cell) is removed, or in a form in which the strain is not removed. The culture product of the *Cutibacterium* sp. strain in the form in which the strain is not removed, may comprise the *Cutibacterium* sp. strain in the culture product.

The culture product may be a whole culture product of the *Cutibacterium* sp. strain, a diluted solution, a concentrate, a dried matter (e.g., lyophilizate, etc.), a lysate, and/or a fraction thereof, and the concentrate may be obtained by centrifuging or evaporating the culture product, and the dried matter may be obtained by drying the culture product using a dryer and the like, and the lyophilizate may be obtained by freeze-drying the culture product using a freeze-dryer and the like, and the lysate may be obtained by physically treating or sonicating the strain or culture product, and the fraction may be obtained by applying the culture product, lysate and the like to a method of centrifugation, chromatography, and the like.

The culture product may be a solid phase (solid, for example, dried matter), a liquid phase (liquid), or a mobile phase, but not limited thereto.

In one embodiment, the culture product may mean a cultured strain obtained by culturing a *Cutibacterium* sp. strain for a certain period of time, a metabolite thereof, and/or a whole medium comprising extra nutrients and the like.

In one embodiment, the culture product may be a cell-free culture product in which a microbial cell (strain) is removed after culturing a *Cutibacterium* sp. strain in a broth. The microbial cell removal may be done by a common method such as separation (for example, centrifugation), filtration and the like, and for example, it may be done by centrifugation. In one specific embodiment, the cell-free culture product may be a supernatant obtained by centrifuging the culture product obtained by culturing a *Cutibacterium* sp. strain in a broth.

In addition, the culture product may be a liquid (or culture filtrate) remained after filtering a culture product obtained by culturing a *Cutibacterium* sp. strain in a broth or a cell-free culture product in which the strain is removed.

In one embodiment, in the culture product, molecular weights of components comprised in the culture product may satisfy a certain numerical range. The culture product may be in a form in which the strain is removed, or in a form in which the strain is not removed.

For example, the culture product may have a molecular weight of the components comprised in the culture product of 10kDa or less, 9kDa or less, 8kDa or less, 7kDa or less, 6kDa or less, 5kDa or less, 4kDa or less, 3kDa or less, 2.5kDa or less, 2kDa or less, 1.5kDa or less, 1kDa or less, 0.5kDa or less, but not limited thereto. In the present description, kDa means kilodalton, and dalton means an atomic mass unit (g/mol). In addition, in the present description, "or less" should be interpreted as including a range exceeding 0. For example, 3kDa or less may mean a numerical range of over 0 to 3 kDa or less.

More specifically, in the culture product, the molecular weight of the components comprised in the culture product may satisfy a range of 10kDa or less, 9kDa or less, 8kDa or less, 7kDa or less, 6kDa or less, 5kDa or less, 4kDa or less, 3kDa or less, 2.5kDa or less, 2kDa or less, 1.5kDa or less, 1kDa or less, 0.5kDa or less, or

0.5 to 4kDa, 0.5 to 3.75kDa, 0.5 to 3.5kDa, 0.5 to 3.25kDa, 0.5 to 3kDa, 0.5 to 2.75kDa, 0.5 to 2.5kDa, 0.5 to 2.25kDa, 0.5 to 2kDa, 0.5 to 1.75kDa, 0.5 to 1.5kDa, 0.5 to 1.25kDa, 0.5 to 1kDa, 0.5 to 0.75kDa, 0.75 to 4kDa, 0.75 to 3.75kDa, 0.75 to 3.5kDa, 0.75 to 3.25kDa, 0.75 to 3kDa, 0.75 to 2.75kDa, 0.75 to 2.5kDa, 0.75 to 2.25kDa, 0.75 to 2kDa, 0.75 to 1.75kDa, 0.75 to 1.5kDa, 0.75 to 1.25kDa, 0.75 to 1kDa, 1 to 4kDa, 1 to 3.75kDa, 1 to 3.5kDa, 1 to 3.25kDa, 1 to 3kDa, 1 to 2.75kDa, 1 to 2.5kDa, 1 to 2.25kDa, 1 to 2kDa, 1 to 1.75kDa, 1 to 1.5kDa, 1 to 1.25kDa, 1.25 to 4kDa, 1.25 to 3.75kDa, 1.25 to 3.5kDa, 1.25 to 3.25kDa, 1.25 to 3kDa, 1.25 to 2.75kDa, 1.25 to 2.5kDa, 1.25 to 2.25kDa, 1.25 to 2kDa, 1.25 to 1.75kDa, 1.25 to 1.5kDa, 1.5 to 4kDa, 1.5 to 3.75kDa, 1.5 to 3.5kDa, 1.5 to 3.25kDa, 1.5 to 3kDa, 1.5 to 2.75kDa, 1.5 to 2.5kDa, 1.5 to 2.25kDa, 1.5 to 2kDa, 1.5 to 1.75kDa, 1.75 to 4kDa, 1.75 to 3.75kDa, 1.75 to 3.5kDa, 1.75 to 3.25kDa, 1.75 to 3kDa, 1.75 to 2.75kDa, 1.75 to 2.5kDa, 1.75 to 2.25kDa, 1.75 to 2kDa, 2 to 4kDa, 2 to 3.75kDa, 2 to 3.5kDa, 2 to 3.25kDa, 2 to 3kDa, 2 to 2.75kDa, 2 to 2.5kDa, 2 to 2.25kDa, 2.25 to 4kDa, 2.25 to 3.75kDa, 2.25 to 3.5kDa, 2.25 to 3.25kDa, 2.25 to 3kDa, 2.25 to 2.75kDa, 2.25 to 2.5kDa, 2.5 to 4kDa, 2.5 to 3.75kDa, 2.5 to 3.5kDa, 2.5 to 3.25kDa, 2.5 to 3kDa, 2.5 to 2.75kDa, 2.75 to 4kDa, 2.75 to 3.75kDa, 2.75 to 3.5kDa, 2.75 to 3.25kDa, 2.75 to 3kDa, 3 to 4kDa, 3 to 3.75kDa, 3 to 3.5kDa, 3 to 3.25kDa, 3.25 to 4kDa, 3.25 to 3.75kDa, 3.25 to 3.5kDa, 3.5 to 4kDa, 3.5 to 3.75kDa, or 3.75 to 4kDa, but not limited thereto.

In one embodiment, to make the molecular weights of the components comprised in the culture product satisfy the numerical range, an appropriate means, procedure, process and the like may be performed, and for example, a filtration or separation process, or the like may be performed. In the present description, "filtration" may be interchangeably used with "separation", and may mean all kinds of processes performed to obtain components comprised in the culture product in a size or molecular weight in a certain numerical range.

In one embodiment, the culture product may be a culture product obtained by filtering a culture product obtained by culturing the *Cutibacterium* sp. strain using a filter to 0.3µm or less, 0.29µm or less, 0.28µm or less, 0.27µm or less, 0.26µm or less, 0.25µm or less, 0.24µm or less, 0.23µm or less, 0.22µm or less, 0.21µm or less, or 0.2µm or less, but not limited thereto. In one specific embodiment, the culture product may be a culture product obtained by filtering a culture product obtained by culturing the *Cutibacterium* sp. strain using a filter to 0.22µm. In the present description, "or less" should be interpreted as including a range exceeding 0 (for example, "0.3µm or less" means a numerical range of over 0 to 0.3µm or less). Any one commonly used for filtering the culture product may be used as the filter without limitation. The culture product may be in a form in which the *Cutibacterium* sp. Strain is removed, or in a form in which it is not removed.

In one embodiment, the culture product may be a filtrate obtained by filtering a culture product obtained by culturing a Cutibacterium sp. strain using a filter to 10kDa or less, 9kDa or less, 8kDa or less, 7kDa or less, 6kDa or less, 5kDa or less, 4kDa or less, 3kDa or less, 2.5kDa or less, 2kDa or less, 1 .5kDa or less, 1kDa or less, 0.5kDa or less, but not limited thereto. In one specific embodiment, the culture product may be a culture product obtained by filtering a Cutibacterium sp. strain using a filter to 3kDa or less. Any one commonly used for filtering the culture product may be used as the filter without limitation.

In the present description, a culture product obtained by filtering to a certain numerical value of kDa or less may be interchangeably used with a separate obtained by separating to a certain numerical value of kDa. In other words, the culture product obtained by filtering (or separate obtained by separating) to a certain numerical value of kDa, may mean that a substance comprised in the culture product has a molecular weight of a certain numerical value of kDa.

In one embodiment, the culture product obtained by filtering to 3kDa or less, may mean that a substance comprised in the culture product has a molecular weight of 3kDa or less. In other words, in the present description, the culture product obtained by filtering to a certain numerical range (kDa), may mean that all the molecular weights of the substances comprised in the culture product satisfy the certain numerical range (kDa).

More specifically, the culture product may be a culture product obtained by filtering a culture product obtained by culturing a *Cutibacterium* sp. strain using a filter to 10kDa or less, 9kDa or less, 8kDa or less, 7kDa or less, 6kDa or less, 5kDa or less, 4kDa or less, 3kDa or less, 2.5kDa or less, 2kDa or less, 1.5kDa or less, 1kDa or less, 0.5kDa or less, or

0.5 to 4kDa, 0.5 to 3.75kDa, 0.5 to 3.5kDa, 0.5 to 3.25kDa, 0.5 to 3kDa, 0.5 to 2.75kDa, 0.5 to 2.5kDa, 0.5 to 2.25kDa, 0.5 to 2kDa, 0.5 to 1.75kDa, 0.5 to 1.5kDa, 0.5 to 1.25kDa, 0.5 to 1kDa, 0.5 to 0.75kDa, 0.75 to 4kDa, 0.75 to 3.75kDa, 0.75 to 3.5kDa, 0.75 to 3.25kDa, 0.75 to 3kDa, 0.75 to 2.75kDa, 0.75 to 2.5kDa, 0.75 to 2.25kDa, 0.75 to 2kDa, 0.75 to 1.75kDa, 0.75 to 1.5kDa, 0.75 to 1.25kDa, 0.75 to 1kDa, 1 to 4kDa, 1 to 3.75kDa, 1 to 3.5kDa, 1 to 3.25kDa, 1 to 3kDa, 1 to 2.75kDa, 1 to 2.5kDa, 1 to 2.25kDa, 1 to 2kDa, 1 to 1.75kDa, 1 to 1.5kDa, 1 to 1.25kDa, 1.25 to 4kDa, 1.25 to 3.75kDa, 1.25 to 3.5kDa, 1.25 to 3.25kDa, 1.25 to 3kDa, 1.25 to 2.75kDa, 1.25 to 2.5kDa, 1.25 to 2.25kDa, 1.25 to 2kDa, 1.25 to 1.75kDa, 1.25 to 1.5kDa, 1.5 to 4kDa, 1.5 to 3.75kDa, 1.5 to 3.5kDa, 1.5 to 3.25kDa, 1.5 to 3kDa, 1.5 to 2.75kDa, 1.5 to 2.5kDa, 1.5 to 2.25kDa, 1.5 to 2kDa, 1.5 to 1.75kDa, 1.75 to 4kDa, 1.75 to 3.75kDa, 1.75 to 3.5kDa, 1.75 to 3.25kDa, 1.75 to 3kDa, 1.75 to 2.75kDa, 1.75 to 2.5kDa, 1.75 to 2.25kDa, 1.75 to 2kDa, 2 to 4kDa, 2 to 3.75kDa, 2 to 3.5kDa, 2 to 3.25kDa, 2 to 3kDa, 2 to 2.75kDa, 2 to 2.5kDa, 2 to 2.25kDa, 2.25 to 4kDa, 2.25 to 3.75kDa, 2.25 to 3.5kDa, 2.25 to 3.25kDa, 2.25 to 3kDa, 2.25 to 2.75kDa, 2.25 to 2.5kDa, 2.5 to 4kDa, 2.5 to 3.75kDa, 2.5 to 3.5kDa, 2.5 to 3.25kDa, 2.5 to 3kDa, 2.5 to 2.75kDa, 2.75 to 4kDa, 2.75 to 3.75kDa, 2.75 to 3.5kDa, 2.75 to 3.25kDa, 2.75 to 3kDa, 3 to 4kDa, 3 to 3.75kDa, 3 to 3.5kDa, 3 to 3.25kDa, 3.25 to 4kDa, 3.25 to 3.75kDa, 3.25 to 3.5kDa, 3.5 to 4kDa, 3.5 to 3.75kDa, or 3.75 to 4kDa, but not limited thereto.

In one embodiment, the culture product obtained by culturing to the range may have excellent (1) preventing, alleviating and/or treating effects of inflammatory skin disease, and/or (2) antimicrobial effects against acne-causing bacteria.

In one embodiment, the culture product of the *Cutibacterium* sp. strain may be a culture product obtained by culturing the Cutibacterium sp. strain in a broth for a certain period of time, or a culture product comprising dead cells of the *Cutibacterium* sp. strain obtained by heat-treating (or sterilizing (for example, autoclaving or hot air drying)) the culture product.

In the present description, "culture" means a series of actions which grow a microorganism under an environmental condition adjusted appropriately artificially. The culture may use any culture condition and culture method known in the art. For example, the culture may be performed continuously in a known batch culture method, continuous culture method, fed batch culture method, batch process or fed batch or repeated fed batch process. Then, the culture condition is, not particularly limited thereto, but may adjust appropriate pH (for example, pH 5 to 9, pH 6 to 8, or pH 6.8) using a basic compound (e.g.: sodium hydroxide, potassium hydroxide or ammonia) or an acidic compound (e.g.: phosphoric acid or sulfuric acid). In one embodiment, air bubble formation can be inhibited using an antifoaming agent such as fatty acid polyglycol ester, and/or an aerobic condition can be maintained by introducing oxygen or an oxygen-containing gas mixture into a culture product.

In one embodiment, the culture temperature may be 20 to 45 °C, 25 to 40 °C, 30 to 40 °C, 30 to 35 °C, or 35 to 40 °C, and the culture condition may be 100 to 500 rpm, 150 to 300 rpm, 150 to 250 rpm, or 200 rpm, the culture time (period) may be 1 to 160 hours, 10 to 100 hours, 12 to 72 hours, 24 to 72 hours, 30 to 60 hours, 40 to 50 hours, 45 to 50 hours, or 48 hours. In one embodiment, the culture may be performed at the culture temperature in the above range for the culture time in the above range.

The broth used in the culture should satisfy requirements of a specific strain in an appropriate way, and those skilled in the art may use appropriately according to the known contents.

In order to culture the *Cutibacterium* sp. strain, survival requirements of a specific strain may be satisfied in an appropriate way by adjusting the temperature, pH and the like under an anaerobic condition in a common broth containing suitable carbon sources, nitrogen sources, amino acids, vitamins and the like. As the carbon sources of supply which may be used in the broth, sugars and carbohydrates (for example, glucose, sucrose, lactose, fructose, maltose, molasses, starch and cellulose), oils and fats (for example, soybean oil, sunflower seed oil, peanut oil and coconut oil), fatty acids (for example, palmitic acid, stearic acid, and linolenic acid), alcohols (for example, glycerol and ethanol) and organic acids (for example, acetic acid) and the like may be individually used or used in combination, but not limited thereto. As the nitrogen source of supply, nitrogen-containing organic compounds (e.g.: peptone, yeast extract, meat juice, malt extract, corn steep liquid, soybean meal powder and urea), or inorganic compounds (e.g.: ammonium sulfate, ammonium chloride, ammonium phosphate, ammonium carbonate and ammonium nitrate) and the like may be individually used or used in combination, but not limited thereto. As the phosphorus source of supply, potassium dihydrogen phosphate, dipotassium dihydrogen phosphate, sodium-containing salts corresponding thereto, and the like may be individually used or used in combination, but not limited thereto. In addition, the culture broth may contain other metal salts (for example, magnesium sulfate or iron sulfate) required for growth and/or comprise essential growth substances such as amino acids and vitamins, but not limited thereto.

### Cutibacterium sp. strain and/or effects of culture product thereof

In one embodiment, the Cutibacterium sp. strain and/or culture product of the strain may have at least one (1kind, 2 kinds, 3 kinds, 4 kinds, or 5 kinds all) activity selected from the group consisting of anti-inflammatory activity, antibacterial activity, skin regeneration activity, skin moisturizing activity, and inflammatory skin disease, or may have the intensified activity. The activity intensification may include all of showing activity that it does not originally have, or showing improved activity compared to the intrinsic activity or activity before modification.

In the present description, the term "anti-inflammatory activity" may mean all activities which reduce inflammation such as inhibiting occurrence of inflammation, or alleviating the level of inflammation, and the like. In one embodiment, the anti-inflammatory activity may mean activity such as inhibiting proliferation of inflammatory cells (for example, macrophage, etc.) causing inflammation, or inhibition of gene transcription or inhibition of protein translation, and the like, of inflammatory substances (for example, inflammatory cytokine, histamine, etc.) generated by inflammatory cells. In one embodiment, the anti-inflammatory activity may mean reducing inflammatory substances (for example, inflammatory cytokine (interleukin-6, etc.), chemokine ligands (CCL2 (C-C Motif Chemokine Ligand 2, chemokine ligand 2), etc.), tumor necrosis factors (TNF-α (tumor necrosis factor alpha), etc.) and the like generated by an immune response. The inflammation may be caused by inflammatory skin disease.

In the present description, the term "antibacterial activity" may mean activity such as inhibiting and/or killing growth of bacteria causing inflammation, and the like. The bacteria causing inflammation may be bacteria causing inflammatory skin disease.

In the present description, the term "skin" may be a concept including not only faces, but also scalp and the entire body. In the present description, the term "skin improving activity" may be a concept including all of skin regeneration, skin moisturizing, skin whitening, skin elasticity improvement, skin wrinkle improvement, skin aging prevention, skin moisture supply, skin nutrition supply, skin tone improvement, skin pore reduction, skin texture improvement, skin peeling, skin volume improvement, scalp protection, prevention of sebum of scalp or skin, hair loss prevention, hair moisture supply, hair nutrition supply, hair volume improvement, skin protection (for example, for skin inflammation alleviation or improvement, skin itchiness improvement or atopy improvement), or skin condition improvement, or the like.

The skin improvement may be at least one selected from the group consisting of skin wrinkle improvement, skin elasticity improvement, skin aging prevention, skin moisturizing improvement, skin moisture supply, and skin nutrition supply.

The skin improvement may be at least one selected from the group consisting of scalp protection, hair loss prevention, hair moisture supply, and hair nutrition supply.

The skin improvement may mean sensitive skin improvement. The sensitive skin improvement, may improve skin responses (for example, skin trouble, immune response, inflammatory response, etc.) caused by temperature (for example, high temperature or low temperature), wind, harmful substances (for example, fine dust, sebum, oil, bacteria, etc.), chemical substances (for example, hormone, etc.).

In the present description, the term "skin regeneration activity" and "skin moisturizing activity" may mean all activities which maintain skin conditions in a healthy state or improve or enhance worsen skin conditions caused by disease. The "skin regeneration activity" and "skin moisturizing activity" may promote proliferation of cells present in the epidermis, dermis, and/or subcutaneous tissue of skin or induce growth of them. The disease which worsens skin conditions may be inflammatory skin disease. In one embodiment, the "skin regeneration activity" and "skin moisturizing activity" may mean increasing the expression level of a skin regeneration-related marker (for example, protein or gene of Src or MMP2 (metalloproteinase-2) or the like), or increasing the expression level of a skin moisturizing-related marker (for example, protein or gene of HAS3 (Hyaluronic acid Synthase 3) or AQP3 (Aquaporin 3), or the like), and the like, respectively.

In one embodiment, the *Cutibacterium* sp. strain and/or culture product of the strain may not comprise at least one (1 kind or 2 kinds) selected from the group consisting of
(a) an Lrp/AsnC family transcriptional regulator (NCBI Reference Sequence: WP_002531510.1) and/or a gene encoding the same, and
(b) a lactococcin 972 family bacteriocin (NCBI Reference Sequence: WP_070651130.1) and/or a gene encoding the same, and/or
may have the activity excellent, compared to the strain (for example, *Cutibacterium* sp. strain) and/or culture product thereof comprising at least one (1 kind, 2 kinds, 3 kinds, 4 kinds, 5 kinds, or 6 kinds) protein selected from the group consisting of the following (1) to (6) or a gene encoding the same:
(1) beta-glucuronidase (NCBI Reference Sequence: WP_002518535.1) or a gene encoding the same,
(2) 2-isopropylmalate synthase (NCBI Reference Sequence: WP_142263178.1) or a gene encoding the same,
(3) 3-isopropylmalate dehydratase (NCBI Reference Sequence: WP_002513330.1) or a gene encoding the same,
(4) type I-E CRISPR-associated protein Cse1/CasA (NCBI Reference Sequence: WP_002514606.1) or a gene encoding the same,
(5) type I-E CRISPR-associated protein Cas7/Cse4/CasC (NCBI Reference Sequence: WP_002514608.1) or a gene encoding the same, and
(6) CRISPR-associated helicase/endonuclease Cas3 (NCBI Reference Sequence: WP_002514605.1) or a gene encoding the same.

### Pharmaceutical composition comprising Cutibacterium sp. strain and/or culture product thereof

Another aspect provides a pharmaceutical composition for preventing, alleviating and/or treating inflammatory skin disease comprising a *Cutibacterium* sp. strain, a culture product of the strain, or a combination thereof. Other embodiment provides a method for preventing, alleviating, and/or treating inflammatory skin disease comprising administering an effective dose of a *Cutibacterium* sp. strain, a culture product of the strain, or a combination thereof into a subject in need of preventing, alleviating, and/or treating inflammatory skin disease.

In the present application, "prevention" means all actions of inhibiting or delaying occurrence of disease (illness) by administration of the composition according to one embodiment, and "treatment" may mean all actions in which symptoms of suspected and outbreak subjects of disease by administration of the composition according to one embodiment are improved or beneficially changed, and "improvement" may mean all actions of at least reducing parameters related to a condition in which the disease is treated by administration of the composition according to one embodiment, for example, the degree of symptoms. The disease may mean inflammatory skin disease.

In the present description, "inflammatory skin disease" may be at least one selected from the group consisting of hair loss, rosacea, skin redness, denticles, blisters, hives, itchiness, skin peeling, rash, allergic drug rash, non-allergic drug rash (for example, drug rash caused by antipsychotics, tetracycline, sulfa drug, antibiotics, hydrochlorothiazide, or non-steroidal anti-inflammatory drug (NSAID)), Stevens-Johnson syndrome, toxic epidermal necrolysis, erythema nodosum, erythema multiforme, keratosis pilaris, psoriasis, eczema, bacterial dermatitis (for example, dermatitis caused by *Cutibacterium* sp. bacteria (for example, *Cutibacterium acnes, Cutibacterium avidum,* and *Cutibacterium granulosum)),* viral dermatitis, atopic dermatitis, rosacea dermatitis, and acne.

The pharmaceutical composition according to one embodiment has no limitation on the method of administration as long as it can reach a target tissue. For example, it may be parenteral administration such as abdominal administration, abdominal injection, intra-articular injection, intra-arterial injection, intravenous injection, dermal administration (for example, application to scalp or skin), dermal injection, local application, intramuscular administration and intraperitoneal administration, or oral administration, but not limited thereto. In addition, the pharmaceutical composition may be administered by any device in which active materials can move to a target cell.

The pharmaceutical composition according to one embodiment may further comprise an appropriate carrier, excipient or diluent commonly used for preparation of a pharmaceutical composition. Specifically, the pharmaceutical composition, may be used by being formulated into a form of oral formulations such as powder, granules, tablets, capsules, suspension, emulsion, syrup, aerosols and the like, external preparations, suppositories, and sterilized injection solution according to a common method, respectively. The carrier, excipient and diluent that may be comprised in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils. When formulated, it is prepared using a diluent or excipient such as a filler, a thickener, a binder, a wetting agent, a disintegrating agent, a surfactant and the like. Solid preparations for oral administration include tablets, pills, powder, granules, capsules and the like, and these solid preparations are prepared by mixing at least one excipient, for example, starch, calcium carbonate, sucrose or lactose, gelatin and the like. In addition, in addition to the simple excipient, lubricants such as magnesium stearate and talc are used. Suspension, oral liquids, emulsion, syrup and the like correspond to liquid preparations for oral administration, and in addition to water, liquid paraffin which are commonly used simple diluents, various excipients, for example, wetting agents, sweeteners, flavoring agents, preservatives, and the like may be comprised. In preparations for parenteral administration, sterilized aqueous solution, non-aqueous solvents, suspension, emulsion, freeze-drying agents, and suppositories are included. As the non-aqueous solvents and suspension, propylene glycol, polyethylene glycol, vegetable oils such as olive oils, injectable esters such as ethyl oleate and the like may be used. As the base of suppositories, witepsol, macrogol, tween 61, cacao butter, laurin butter, glycerogelatin and the like may be used.

The pharmaceutical composition according to one embodiment may be administered in a pharmaceutically effective dose, and in the present application, "pharmaceutically effective dose" may refer to an amount sufficient for treating or preventing disease at a reasonable benefit/risk ratio applicable to medical treatment or prevention, and the effective dose level may be determined according to factors including the severity of disease, activity of a drug, a patient's age, body weight, health, gender, a patient's sensitivity to a drug, administration hours, administration routes, and excretion ratios of the used composition of the present application, treatment period, a drug used in combination or simultaneously with the used composition of the present application and other factors well known in the medical field. The pharmaceutical composition according to one embodiment may be administered as an individual therapeutic agent or administered as use in combination with another therapeutic agent, and it may be administered sequentially or simultaneously with a conventional therapeutic agent. In addition, it may be administered single or multiple times. In consideration of all the above factors, it is important to administer an amount capable of obtaining the maximal effect with a minimal amount without side effects.

The dosage of the pharmaceutical composition according to one embodiment (based on the dry matter weight of the culture product of the *Cutibacterium* sp. strain) may be administered into a mammal by about 0.0001 to 10000mg/kg, 0.0001 to 5000mg/kg, 0.0001 to 1000mg/kg, 0.0001 to 900mg/kg, 0.0001 to 800mg/kg, 0.0001 to 700mg/kg, 0.0001 to 600mg/kg, 0.0001 to 500mg/kg, 0.0001 to 400mg/kg, 0.0001 to 300mg/kg, 0.0001 to 200mg/kg, 1 to 1000mg/kg, 1 to 900mg/kg, 1 to 800mg/kg, 1 to 700mg/kg, 1 to 600mg/kg, 1 to 500mg/kg, 1 to 450mg/kg, 1 to 400mg/kg, 1 to 350mg/kg, 1 to 300mg/kg, 1 to 250mg/kg, 10 to 1000mg/kg, 10 to 900mg/kg, 10 to 800mg/kg, 10 to 700mg/kg, 10 to 600mg/kg, 10 to 500mg/kg, 10 to 450mg/kg, 10 to 400mg/kg, 10 to 350mg/kg, 10 to 300mg/kg, 10 to 250mg/kg, 100 to 1000mg/kg, 100 to 900mg/kg, 100 to 800mg/kg, 100 to 700mg/kg, 100 to 600mg/kg, 100 to 500mg/kg, 100 to 450mg/kg, 100 to 400mg/kg, 100 to 350mg/kg, 100 to 300mg/kg, 100 to 250mg/kg, 200 to 1000mg/kg, 200 to 900mg/kg, 200 to 800mg/kg, 200 to 700mg/kg, 200 to 600mg/kg, 200 to 500mg/kg, 200 to 450mg/kg, 200 to 400mg/kg, 200 to 350mg/kg, 200 to 300mg/kg, or 200 to 250mg/kg for one day, but not limited thereto. The administration frequency of the pharmaceutical composition of the present application is not particularly limited thereto, but it may be administered once a day or administered several times by dividing the dose. The dosage cannot limit the range of the present application in any side.

The administration subject of the pharmaceutical composition provided in the present description may be a mammal including humans, dogs, cats, horses, cattle, pigs, goats, rabbits, mice, rats, and the like or a cell or tissue isolated therefrom, or a culture product thereof, and in one embodiment, the subject may be a subject (mammal such as humans, etc.) which needs prevention, alleviation and/or treatment of inflammatory skin disease, and is with inflammatory skin disease as described above, or a cell or tissue isolated therefrom, or a culture product thereof.

The pharmaceutical composition according to one embodiment may comprise the *Cutibacterium* sp. strain, a culture product of the strain, or a combination thereof by 1 to 80% by weight, 5 to 80% by weight, 5 to 75% by weight, 5 to 70% by weight, 5 to 65% by weight, 50 to 70% by weight, 55to 65% by weight, 60 to 65% by weight, 10 to 60% by weight, 15 to 60 % by weight, 20 to 60 % by weight, 1 to 50% by weight, 5 to 50% by weight, 10 to 50% by weight, 15 to 50 % by weight, 20 to 50 % by weight, 1 to 40% by weight, 5 to 40% by weight, 10 to 40% by weight, 15 to 40 % by weight, 20 to 40 % by weight, 1 to 30% by weight, 5 to 30% by weight, 10 to 30% by weight, 15 to 30 % by weight, 20 to 30 % by weight, 1 to 25% by weight, 5 to 25% by weight, 10 to 25% by weight, 15 to 25% by weight, 20 to 25% by weight, or 23 to 25% by weight.

### Food composition comprising Cutibacterium sp. strain and/or culture product thereof

Other aspect provides a food composition for preventing and/or alleviating inflammatory skin disease comprising a *Cutibacterium* sp. strain, a culture product of the strain, or a combination thereof. The food composition may be a food itself, or in a food additive form added to food, or a health functional food.

The food composition according to one embodiment may mean meat, sausages, bread, chocolate, candies, snacks, confectionery, pizza, ramen, other noodles, gum, dairy products including ice cream, various kinds of soups, beverages, tea, drinks, alcoholic beverages, vitamin complexes, health functional foods and health foods and the like, and includes all foods in the conventional sense.

The health functional food is the same term with food for special health use (FoSHU), and means a food with high medical, and medicinal effects, processed to effectively show a body modulation function in addition to nutrition supply. Herein, "function" means regulating nutrients for the human structure and function or obtaining useful effects for a health use such as a physiological action and the like. The food of the present application may be prepared by a method commonly used in the art, and during the preparing, it may be prepared by adding a raw material and a component commonly added in the art. In addition, the formulation of the food may be also prepared without limitation as long as it is a formulation admitted as a food. The food composition of the present application may be prepared in various forms of formulations, and unlike a general drug, it has an advantage of no side effects which may occur during long-term use of a drug, as it uses a food as a raw material, and it has excellent portability, so the food of the present application can be ingested as an adjuvant for enhancing the effect of preventing or alleviating inflammatory skin disease.

The health food refers to a food having an active effect of maintaining or enhancing health compared to a common food, and the health supplement food means a food for a health supplement purpose. In some cases, the terms of health functional food, health food, and health supplement food can be interchangeably used.

Specifically, the health functional food is a food in which the composition according to one embodiment is added to a food material such as beverages, tea, spices, gum, confectionery, and the like, or is prepared by capsulation, powdering, suspension and the like, and refers to one which brings a specific effect on the health when it is ingested, but it has an advantage of having no side effects which may occur during long-term use of a drug as it uses a food as a raw material unlike a general drug.

The food composition according to one embodiment is possible to be ingested for everyday use, so a high effect for preventing or alleviating inflammatory skin disease can be expected, and thus, it can be very usefully used.

The food composition may further comprise a physiologically acceptable carrier, and the type of the carrier is not particularly limited, and any carrier commonly used in the art may be used.

In addition, the food composition may comprise an additional component capable of improving smell, taste, vision and the like as commonly used in a food composition. For example, it may comprise vitamins A, C, D, E, B1, B2, B6, B12, niacin, biotin, folate, pantothenic acid and the like. In addition, it may comprise a mineral such as zinc (Zn), iron (Fe), calcium (Ca), chrome (Cr), magnesium (Mg), manganese (Mn), copper (Cu), chrome (Cr) and the like. In addition, it may comprise an amino acid such as lysine, tryptophane, cysteine, valine and the like.

In addition, the food composition may comprise food additives such as preservatives (potassium sorbate, sodium benzoate, salicylic acid, sodium dehydrocitrate, etc.), sterilizers (bleaching powder and highest bleaching powder, sodium hypochlorite, etc.), antioxidants (butylhydroxyanizole (BHA), butylhydroxytoluene (BHT), etc.), coloring agents (tar color, etc.), color formers (sodium nitrite, etc.), bleaching agents (sodium sulfite), seasonings (MSG sodium glutamate, etc.), sweeteners (dulcin, cyclamate, saccharin, sodium, etc.), flavoring agents (vanillin, lactones, etc.), inflating agents (alum, potassium D-bitartrate, etc.), reinforcing agents, emulsifiers, thickeners (thickening agents), coating agents, gum bases, antifoaming agents, solvents, improving agents, and the like. The additive may be selected depending on the type of food, and may be used in an appropriate amount.

The composition according to one embodiment may be added as it is or used with other food or food component, and may be appropriately used according to a common method. The mixed amount of active ingredients may be properly determined according to the purpose of use thereof (prevention, health or therapeutic treatment). In general, during preparation of a food or beverage, the food composition of the present application may be added in an amount of 50 parts by weight or less, specifically, 20 parts by weight or less based on the food or beverage. However, when it is ingested during a long period of time for a purpose of health and hygiene, the content below the above range may be comprised, and as there is not any problem in the aspect of safety, the active ingredients may be used even in an amount over the above range.

As one embodiment of the food composition, it may be used as a health beverage composition, and in this case, as same as a common beverage, various flavoring agents or natural carbohydrates or the like may be contained as additional components. The afore-mentioned natural carbohydrates may be monosaccharides such as glucose and fructose; disaccharides such as maltose and sucrose; polysaccharides such as dextrin and cyclodextrin; or sugar-alcohols such as xylitol, sorbitol, erythritol and the like. As the sweeteners, natural sweeteners such as thaumatin and stevia extract; synthetic sweeteners such as saccharin and aspartame and the like may be used. The ratio of the natural carbohydrates may be generally about 0.01 to 0.04 g, specifically, about 0.02 to 0.03 g, per 100 mL of the health beverage composition of the present application.

In addition thereto, the health beverage composition may contain various nutrients, vitamins, electrolytes, flavors, coloring agents, pectic acid, salts of pectic acid, alginic acid, salts of alginic acid, organic acids, protective colloidal thickeners, pH adjusting agents, stabilizers, preservatives, glycerin, alcohols or carbonating agents, or the like. In addition, flesh for preparation of a natural fruit juice, a fruit juice beverage, or a vegetable beverage may be contained. These components may be used independently or as mixed. The ratio of these additives is not significantly important, but it is common to be selected in a range of 0.01 to 0.1 part by weight per 100 parts by weight of the health beverage composition of the present application.

The food composition according to one embodiment may be comprised by various % by weights as long as it can exhibit an effect of preventing or alleviating inflammatory skin disease, but for example, the composition according to one embodiment may be comprised by 0.00001 to 100 % by weight or 0.01 to 80 % by weight compared to the total weight of the food composition, but not limited thereto.

### Feed composition comprising Cutibacterium sp. strain and/or culture product thereof

Other aspect provides a feed composition for preventing and/or alleviating inflammatory skin disease comprising a *Cutibacterium* sp. strain, a culture product of the strain, or a combination thereof.

In the present description, "feed" may mean any natural or artificial diet, meal, or the like or a component of the meal, which is for an animal to eat, ingest or digest, or is suitable therefor.

In the present description, the feed composition for preventing and/or alleviating inflammatory skin disease comprising a *Cutibacterium* sp. strain, a culture product of the strain, or a combination thereof may be used as the feed itself, or may be used in a feed additive form which is added to feed.

The type of the feed is not particularly limited, and feed commonly used in the art may be used. Non-restrictive examples of the feed may include vegetable feed such as grains, root fruits, food processed by-products, algae, fibers, pharmaceutical by-products, fat and oils, starch, meals or grain by-products and the like; and animal feed such as proteins, inorganic matters, fat and oils, minerals, monocyte proteins, animal planktons, or foods and the like. They may be used alone or used by mixing 2 kinds or more.

The feed composition may further comprise an excipient, a diluent, and/or an additive, and the like. The feed composition may comprise effective components for promoting growth of animals, nutritional components, nutrition supplement components, components increasing preservation stability, coating material components, amino acids, vitamins, enzymes, non-protein nitrogen compounds, silicates, buffers, extracting agents, probiotics for preventing disease and the like; enzymes such as amylase, lipase and the like; vitamins such as L-ascorbic acid, choline chloride, inositol and the like; minerals such as potassium chloride, iron citrate, magnesium oxide, phosphates and the like; amino acids such as lysine, alanine, methionine, and the like; organic acids such as fumaric acid, butyric acid, lactic acid, and the like or salts thereof; antioxidants such as vitamin C, vitamin E and the like, fungicides such as calcium propionate; emulsifiers such as lecithin, glycerin fatty acid ester and the like; and/or pigments and the like, in addition to the above components. Even if not described above, the feed composition according to one embodiment may further comprise other nutrients within the range that can be expected by those skilled in the art.

There is no particular limitation on a subject (animal) to which feed comprising the composition according to one embodiment is fed, but it may be a mammal, a fish, a crustacean and/or a shell, and for example, a pig, cow, horse, goat, deer, sheep, chicken, duck, goose, turkey, dog, cat, rabbit, or fish.

When the feed composition is used in a feed additive form, it may be prepared in a form in which the feed additive according to one embodiment is added to commercially available feed. It is preferable that feed in which the feed additive according to one embodiment is used is a powder or pellet formulation, or a liquid formulation, but not limited thereto. In addition, the added amount of the feed additive of the present application added to feed does not require special limitation.

The feed may be prepared by separately preparing the composition of the present application in a feed additive form and mixing it to feed, or directly adding it during preparation of feed. The feed additive of the present application comprised in the feed may be in a liquid or dried state, and for example, it may be in a dried powder form. The feed additive may be comprised in an amount of 0.005 to 10 % by weight, 0.05 to 10 % by weight, 0.1 to 10 % by weight, 0.005 to 5 % by weight, 0.05 to 5 % by weight, 0.1 to 5 % by weight, 0.005 to 2 % by weight, 0.05 to 2 % by weight, or 0.1 to 2 % by weight of the total feed weight, but not limited thereto. In addition, the feed may further comprise common additives which can increase preservability of feed in addition to the feed additive.

Feed in which the feed additive may be added may be commercially available feed, or selected from the group consisting of grains, root fruits, food processed by-products, algae, fibers, pharmaceutical by-products, fat and oils, starch, meals, grain by-products, proteins, inorganic matters, minerals, monocyte proteins, animal planktons, leftover food, and the like, but not limited thereto.

### Antibacterial composition comprising Cutibacterium sp. strain and/or culture product thereof

Other aspect provides an antibacterial composition against inflammatory skin disease-causing bacterum comprising a *Cutibacterium* sp. strain, a culture product of the strain, or a combination thereof. Other embodiment provides a method for inhibiting causative bacteria of inflammatory skin disease comprising applying an effective dose of a *Cutibacterium* sp. strain, a culture product of the strain, or a combination thereof to a subject in need of antibacterial treatment against inflammatory skin disease-causing bacterum. Other embodiment provides a use of a *Cutibacterium* sp. strain, a culture product of the strain, or a combination thereof for using in preparation of an antibacterial composition against inflammatory skin disease-causing bacterum.

In the present description, "antibacterial composition" may mean a composition having antibacterial activity. The antibacterial activity is same as described above, and may refer to inhibiting and/or killing growth of inflammatory skin disease-causing bacterum. The antibacterial activity may be interchangeably used with antibiotic activity, and the antibacterial composition encompasses all types of agents with growth inhibiting activity and/or killing activity against inflammatory skin disease-causing bacterum, and may be interchangeably used with antibiotic composition, antibacterial agent, antibiotic, preservative, sterilizing agent, and the like, unless otherwise mentioned.

The inflammatory skin disease-causing bacterum may be at least one (1 kind, 2 kinds, 3 kinds, 4 kinds, 5 kinds, or 6 kinds all) selected from the group consisting of *Cutibacterium acnes (C. acnes), Cutibacterium avidum (C. avidum), Cutibacterium granulosum (C. granulosum), Staphylococcus aureus (S. aureus), Bacteroides fragilis (B. fragilis*), and *Staphylococcus epidermidis* (*S. epidermidis*), but not limited thereto.

The inflammatory skin disease is same as described above, and for example, may be disease such as acne or rosacea or the like, but not limited thereto.

Other embodiment provides a disinfectant comprising a *Cutibacterium* sp. strain, a culture product of the strain, or a combination thereof as an active ingredient. Other embodiment provides a use of the *Cutibacterium* sp. strain, culture product of the strain, or combination thereof for using in preparation of a disinfectant. Other embodiment provides a method for disinfection, comprising applying the disinfectant to a subject in need of disinfection. Other embodiment provides a detergent comprising a *Cutibacterium* sp. strain, a culture product of the strain, or a combination thereof as an active ingredient. Other embodiment provides a use of the *Cutibacterium* sp. strain, culture product of the strain, or combination thereof for using in preparation of a detergent. Other embodiment provides a method for cleaning, comprising applying the detergent to a subject in need of cleaning.

The disinfectant refers to a general term for agents to prevent pathogen infection, and may be used for general living disinfectants, disinfectants for food and cooking areas and facilities, disinfection for buildings such as poultry farms and cattle shed and the like, livestock, drinking water, litter, egg trays, transport vehicles, various kinds of growth and development goods such as tableware, and the like.

The antibacterial composition has an antibacterial effect against inflammatory skin disease-causing bacterum, so it may be applied as a use for cleaning (washing) a skin surface or each part of the body of a subject who is exposed or may be exposed to inflammatory skin disease-causing bacterum.

### Cosmetic composition comprising Cutibacterium sp. strain and/or culture product thereof

Other aspect provides a cosmetic composition for preventing and/or alleviating inflammatory skin disease, or alleviating skin, comprising a *Cutibacterium* sp. strain, a culture product of the strain, or a combination thereof.

The cosmetic composition according to one embodiment may be a cosmetic composition or skin external preparation composition, and this cosmetic composition or skin external preparation composition may have an anti-inflammatory, or skin improving activity (for example, effects for promoting skin regeneration activity, and/or strengthening skin moisturizing activity (function)). The skin improving activity is same as described above.

The formulation of the skin external preparation composition or cosmetic composition provided in the present description is not particularly limited, and for example, it may be formulated as solution, suspension (anhydrous and aqueous), emulsion, paste, gel, cream (oil in water emulsion, water in oil emulsion, multiphase, etc.), powder, ointment, patch, cosmetic water, essence, gel, lotion, solution, anhydrous products (oil and glycol-based), mask, pack, powder, or capsule (soft capsule, hard capsule) formulation with coating of gelatin, and the like, or spray, or the like.

The cosmetic composition may be any one formulation selected from the group consisting of solution, suspension, emulsion, paste, gel, cream, powder, ointment, patch, cosmetic water, essence, gel, lotion, mask, pack, powder, capsule and spray.

The skin in the present application is a concept including not only a face, but also scalp and a whole body, and the skin external preparation composition which can be applied to this scalp includes shampoo, rinse, treatment, hair growth solution, and the like, and as a use of a body cleanser which can be applied to the whole body and the like, it may be prepared in various forms.

In one embodiment, the composition comprises a common surfactant (emulsifier), and may have properties of an emulsified or solubilized form of an O/W (oil in water), W/O (water in oil), W/O/W, or O/W/O formulation. The surfactant may be at least one selected from all surfactants commonly used for preparation of a skin external preparation composition and/or a cosmetic composition, and for example, it may be at least one selected from the group consisting of non-ionic surfactants and anionic surfactants, but not limited thereto. The non-ionic surfactants may be at least one selected from the group consisting of polyoxyethylene-based surfactants, polyglycerin-based surfactants, sugar ester-based surfactants and the like, and for example, may be at least one selected from the group consisting of polyglyceryl-methylglucose distearate, polyglyceryl-methylglucose stearate, polyglyceryl-distearate (for example, polyglyceryl-10 distearate, etc.), polyglyceryl-stearate, hydrogenated lecithin, cetearyl olivate, sorbitan olivate, PEG (polyethylene glycol))-stearates (for example, PEG-100 stearate, PEG-40 stearate, etc.), glyceryl stearate, glyceryl stearate SE, decylglucoside, coco-glucoside, laurylglucoside, caprylyl/caprylglucoside, arachidyl glucoside, C12-20 (linear or branched) alkyl glucoside, cetearyl glucoside, ceteareths (for example, ceteareth-20, etc.), steareths (for example, steareth-2, steareth-21, etc.), polyethylene glycol ether of oleyl alcohol (for example, oleth-20, etc.), methylglucose sesquistearate, sorbitan stearate, sucrose cocoate, polysorbates (for example, polysorbate 60, etc.), sorbitan sesquioleate, sorbitan olivate, oil palm oil, oil palm kernel oil, and the like, but not limited thereto. The anionic surfactants may be at least one selected from the group consisting of sodium lauryl sulfate, ammonium lauryl sulfate, magnesium lauryl sulfate, triethanolamine lauryl sulfate, sodium polyoxyethylene lauryl sulfate, ammonium polyoxyethylene lauryl sulfate, sodium cocoamphoacetate, ammonium laureth sulfate, ammonium lauryl sulfate, cocamide MEA, disodium laureth sulfosuccinate, TEAE-cocoylglutamate, sodium cocoyl apple amino acid, cocamide DEA, sodium laureth sulfate, sodium lauryl sulfate, potassium cocoyl glycinate, sodium stearoyl glutamate, potassium cetyl phosphate, and the like, but not limited thereto.

The skin external preparation composition and/or cosmetic composition, may comprise components commonly contained to apply to skin, at least one selected from the group consisting of oil, polymers, thickeners, additives, and the like, in addition to a *Cutibacterium* sp. strain, a culture product of the strain, or a combination thereof. The components commonly contained and contents thereof may be appropriately adjusted according to the function, properties, and the like of a desired composition.

For example, the oil may be at least one selected from the group consisting of silicon oil, for example, at least one selected from the group consisting of cyclopentasiloxane, dimethicone, dimethiconol, cyclomethicone, trisiloxane, amodimethicone, phenyltrimethicone, disiloxane, cyclohexasiloxane, PEG-dimethicones (for example, PEG-12 dimethicone, PEG-10 dimethicone, PEG-7 dimethicone, etc.), polysilicones (for example, polysilicone-11, etc.), simethicone, diphenyldimethicone, PEG-9 polydimethylsiloxyethyldimethicone, and the like; non-silicone oil, for example, at least one selected from the group consisting of caprylic/capric triglyceride, butylene glycol dicaprylate/dicaprate, hydrogenated polyisobutene, isopropyl myristate, cetylethyl hexanoate, hydrogenated polydecene, diethoxyethyl succinate, hexyl laurate, triethyl hexanoin, isononyl isononanoate, polyisobutene, and the like; triglyceride; wax and the like. The polyol may be at least one selected from the group consisting of butylene glycol, glycerin, glycereth (for example, glycereth-26, etc.), methylpropanediol, PEG (polyethylene glycol))/PPG (polypropylene glycol)) copolymers (for example, PEG/PPG-17/6 copolymer, etc.), methyl gluceths (for example, methyl gluceth-20, etc.), dipropylene glycol, propylene glycol, propanediol, caprylyl glycol, pentylene glycol, and the like. The thickener may be at least one selected from polymer compounds consisting of carbomers, ammonium acryloyldimethyl taurate/VP copolymers, xanthan gum, sodium polyacrylate, hydroxyethyl cellulose, acrylate/C10-30 alkylacrylate crosspolymers, sodium polyacrylate, sodium acrylate/sodium acryloyldimethyl taurate copolymers, magnesium aluminum silicate, sodium carbomers, and the like. The additive may comprise at least one selected from the group consisting of ion sequestering agents, emulsion stabilizers, pH adjusting agents, skin conditioning agents, flavoring agents, preservatives, sterilizing agents, oxidation stabilizers, antioxidants, free radical breakers, opacifying agents, stabilizers, emollients, vitamins, insect repellents, preservatives, anti-inflammatory agents, alkalinizing or acidifying agents, coloring agents (pigments), solubilizers, carriers and the like, but not limited thereto.

The composition may comprise a solvent commonly used for preparation of a skin external preparation and/or a cosmetic composition, and the available solvent may be at least one selected from the group consisting of purified water, bedrock water, hot spring water, glacial water, sea water, deep ocean water, plant extracting water and the like, but not limited thereto.

The composition may further comprise an active ingredient which exhibits an advantageous effect for skin care and/or hair/scalp care in addition to the components described above. For example, the advantageous effect may mean skin whitening, skin elasticity improvement, skin wrinkle improvement, skin aging prevention, skin moisture supply, skin moisturizing, skin nutrition supply, skin pore reduction, skin texture improvement, skin peeling, skin volume improvement, scalp protection, prevention of sebum of scalp or skin, hair loss prevention, hair moisture supply, hair nutrition supply, hair volume improvement, and the like, but not limited thereto, and is all desired effects in skin/hair/scalp of a subject for application. For example, the active ingredient may be at least one selected from the group consisting of various kinds of plant extracts (for example, anemarrhena extract, green tea extract, grape extract, lotus extract, tomato extract, lonicera aflower extract, camellia flower extract, seaweed extract, etc.), plant callus culture products or culture extracts, yeast extracts, cell-containing or cell-free yeast culture products or culture extracted, vitamins, various kinds of peptides, lipids, or fatty acids (for example, various kinds of fish and shellfish-derived lipids, fatty acids, peptides, etc.), hyaluronic acid, glycoside (for example, arbutin, etc.), other various kinds of compounds for skin, and the like, but not limited thereto, and depending on the desired effect, an appropriate active ingredient may be selected and used. In one embodiment, the active ingredient may be a mixture of hydrogenated polyisobutene and anemarrhena extract, but not limited thereto.

Other embodiment provides a product comprising the skin external preparation composition or cosmetic composition. The product may be a product for skin, hair, and/or scalp care, and for example, may be all types of cosmetics and/or cleansing products to apply to skin of faces, hands, feet, whole bodies and the like, scalp, hair, and the like. In one embodiment, the product may be at least one selected from the group consisting of cosmetics such as skin, lotion, cream, essence, pack, foundation, color cosmetics, suncream (sunscreen), BB cream, two-way cake, face powder, compact, makeup base, skin cover, eye shadow, lipstick, lip gloss, lip fix, eyebrow pencil, cosmetic water, etc.; and cleansing products such as shampoo, soap, cleansing foam, body cleanser, etc., and the like.

The subject of administration of the composition (pharmaceutical composition, cosmetic composition, food composition, feed composition, and/or antibacterial composition) comprising a Cutibacterium sp. strain, a culture product of the strain, or a combination thereof and method provided in the present description may be a mammal including humans, dogs, cats, horses, cows, pigs, goats, rabbits, mice, rats, and the like or a cell, tissue isolated therefrom, or a culture product thereof, and in one embodiment, the subject may be a subject (mammal such as humans, etc.) which needs prevention, alleviation, and/or treatment of inflammatory skin disease described above, or has inflammatory skin disease, or a cell, tissue isolated therefrom, or a culture product thereof.

### [ADVANTAGEOUS EFFECTS]

The present application relates to a use of a *Cutibacterium* sp. strain, a culture product of the strain or a combination thereof for preventing, alleviating and/or treating inflammatory skin disease, and the composition comprising the Cutibacterium sp. strain, the culture product of the strain or the combination thereof has the excellent effects of preventing, alleviating and/or treating inflammatory skin disease.

### [BRIEF DESCRIPTION OF THE DRAWINGS]

FIG. 1 is the result of performing the hemolytic test to confirm presence or absence of toxicity caused by hemolytic activity of the 6 strains (CJRS-10651, CJRS-10652, CJRS-10653, CJRS-10654, CJRS-10655, and CJRS-10656 strains; same below) selected in the present application.
FIG. 2a and FIG. 2b are graphs shown in a phylogenetic tree form to confirm common genetic characteristics of the 6 strains of which ribotype is RT2 selected in the present application.

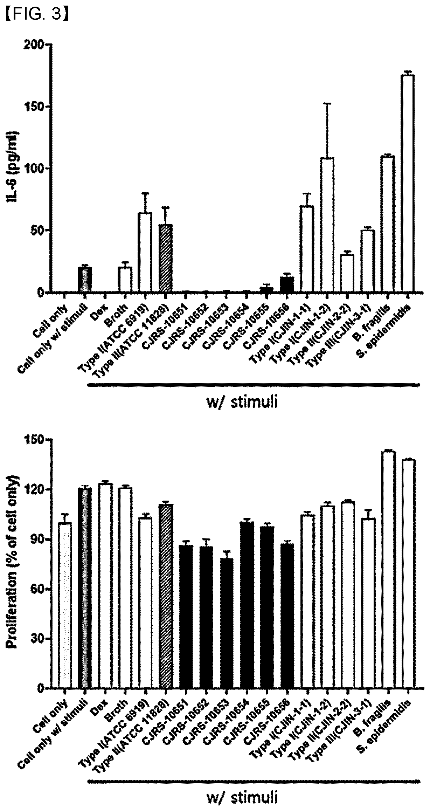

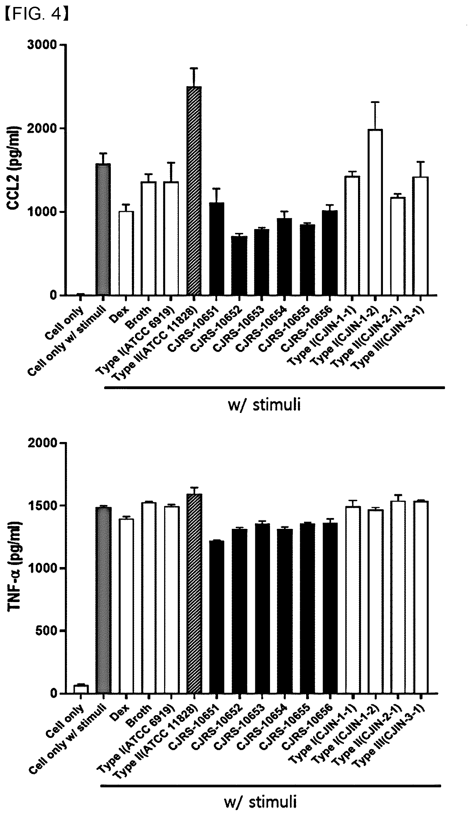

FIG. 3 and FIG. 4 are graphs which confirm the immune response in immunocytes (proliferation of immunocytes, and expression levels of IL-6, CCL2, and TNF-a), compared to the control group (Cell only w/ stimuli; group in which only an inflammatory response is induced), positive control group (group in which an inflammatory response is induced and then Dex is treated), negative control group (group in which a vegetable empty broth is treated after an inflammatory response) and comparative group (group in which the culture products of Type I (ATCC 6919), Type II (ATCC 11828), Type I (CJIN1-1), Type I (CJIN1-2), Type II (CJIN2-1), Type II (CJIN2-2), Type III (CJIN3-1), *B. fragilis* and S. *epidermidis* are treated after an inflammatory response is induced), when the culture products of the 6 strains selected in the present application are treated.
FIG. 5 is a graph which confirms the antimicrobial activity against *C. acnes* (ATCC 6919), which is an acne-causing strain of each of culture products of the 6 strains selected in the present application. (NC: nothing treated, Doxy: group treated with doxycycline as a positive control group)
FIG. 6 is a graph which confirms the inhibitory effect of biofilm formation of the bacteria (*S*. *epidermidis)* of each of the culture products of the 6 strains selected in the present application. (NC: nothing treated, Doxy: group treated with doxycycline as a positive control group)
FIG. 7 is a graph which confirms the cytotoxic effect when each of the culture products of the 6 strains selected in the present application is treated by concentration (5%(v/v), 10%(v/v), 20%(v/v)).
FIG. 8a and FIG. 8b are drawings and graphs which confirm the skin efficacy (skin cell regeneration efficacy), when the culture products of the 6 strains selected in the present application are treated, respectively (0 hour, 4 hours and 8 hours after a wound).
FIG. 9, and FIG. 10 are graphs which confirm the expression level of Src, MMP2 (metalloproteinase-2) that are skin regeneration-related markers, and the expression level of HAS3 (Hyaluronic acid Synthase 3) and AQP3 (Aquaporin 3) that are skin moisturizing-related markers, when the culture product of the 6 strains selected in the present application are treated, respectively.
FIG. 11 shows the secreted amount of the inflammatory cytokine (IL-6) and the anti-inflammatory cytokine (IL-10) secreted by the Raw264.7 macrophage, which is calculated at a ratio of IL-10/IL-6, by culturing the 6 strains (living cells) selected in the present application. (A *B. fragilis* strain was used as a control group.)
FIG. 12 and FIG. 13 are graphs which confirm proliferation of immunocytes and the expression levels of the inflammatory cytokines (IL-6 and IL-8) when the culture products of the 2 strains (CJRS-10652 strain and CJRS-10653 strain) selected in the present application are treated. (Positive control group: group using dexamethasone (Dex))

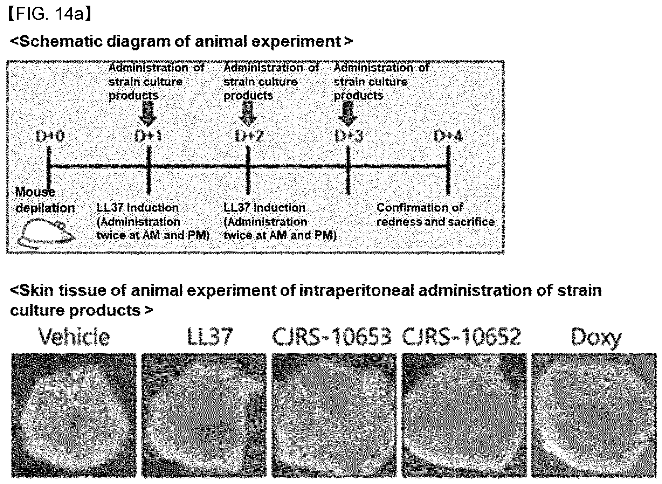

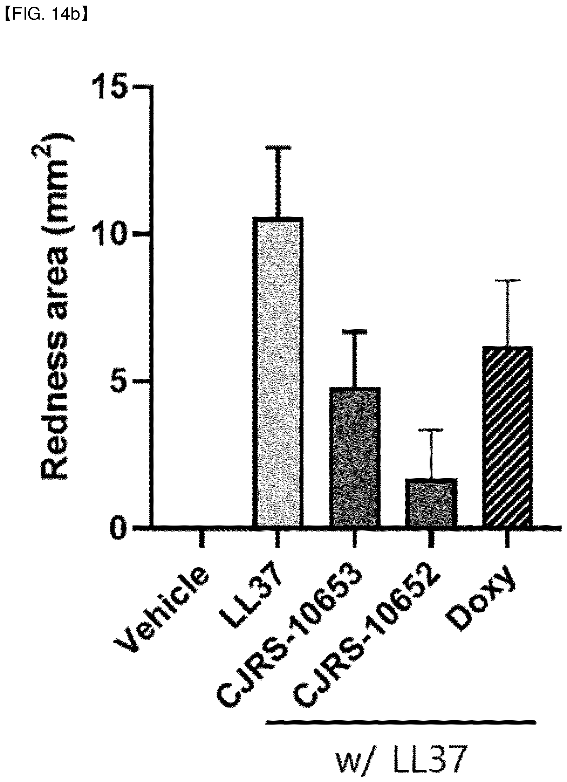

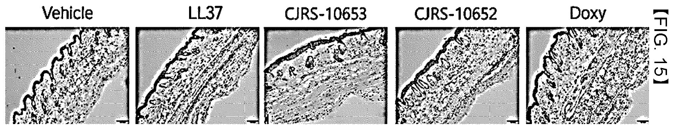

FIG. 14a, FIG. 14b, and FIG. 15 are graphs and photographs which confirm the degree of skin erythema, epithelial tissue hyperplasia, and skin inflammation, when the culture products of the 2 strains (CJRS-10652 strain and CJRS-10653 strain) selected in the present application are intraperitoneally administered into mice in which rosacea is induced, respectively. (Vehicle: control group in which nothing is treated, LL37: group in which LL37 peptide is treated to induce rosacea lesions, Doxy: group in which doxycycline is intraperitoneally administered into mice in which lesions are induced, as a positive control group)
FIG. 16 and FIG. 17 are the results that confirm the expression level of a barrier improvement index, Filaggrin and a moisturizing improvement index, Aquaporin-3, after treating the strain culture products selected in the present application into the skin model T&R HuSkin.
FIG. 18 and FIG. 19 are the results that confirm presence or absence of cytotoxicity and collagen production function after treating the strain culture products selected in the present application into human fibroblasts.

### [MODE FOR INVENTION]

Hereinafter, the present invention will be described in more detail by the following examples. However, they are intended to illustrate the present invention only, but the scope of the present invention is not limited by these examples.

### Experimental example 1. Strain acquisition

### 1) Selection of test subjects

The present test was conducted on 73 healthy adult men and women aged between 18 and 39 years old (CRA Korea company was selected as the consignment organization for human sebum collection, IRB approval (approval number: CRAIRB20-030502)).

### 2) Skin sebum collection

After washing a face with water lightly, a nose was lightly wiped by wetting gauze with sterile water. Sterile gauze soaked in warm water was placed on the nose for about 5 minutes and waited. 1 drop of instant adhesive (Cyanoacrylate) was applied to the tape, and then it was waited for about 10 seconds. After applying the tape to the nose and waiting for 1 minute, sebum was extracted (SSB, Skin Surface Biopsy). By placing the sebum-smeared tape in 1.5ml e-tube (glycerol 50% + RCM (Reinforced Clostridial Medium, BD Difco) 50%), a skin sebum sample was collected.

### 3) Skin strain culture and acquisition

The sebum sample was diluted in PBS (100-103), and then streaked in an RCM agar plate, and then placed in an anaerobic gas pack jar and cultured at 37 °C for 96 hours. 10-20 single colonies per plate were added in an RCM broth, and activated in an anaerobic gas pack jar (37 °C) for 24 hours. After 24 hours, 1% of the sample was inoculated in a fresh RCM broth, and then it was placed on an anaerobic gas pack jar and cultured at 37 °C for 48 hours to acquire a total of 1735 human skin-derived strains. The secured strains were subjected to 16S rRNA and RecA sequencing using the primers listed in Table 1 below, and 166 specific *C*. *acnes* strains were selected based on sequencing. Six specific *C. acnes* strains were selected though a macrophage-based proliferation and inflammatory marker (IL-6, CCL2, TNF-α) inhibition test.

It was confirmed that the selected 6 strains in the present application, CJRS-10651 strain, CJRS-10652 strain, CJRS-10653 strain, CJRS-10654 strain, CJRS-10655 strain, and CJRS-10656 strain comprised 16S rRNA of SEQ ID NO: 8 in common. The sequence type, ribotype, and immunological phenotype of the secured strains were confirmed, and they were shown in Table 2 below.

**[Table 1]**

| 16S and RecA PCR sequencing primer 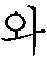 16S rRNA gene sequence | |
|---|---|
| 16S PCR | |
| 27F | AGAGTTTGATCMTGGCTCAG (SEQ ID NO: 1) |
| 1482R | TACGGYTACCTTGTTACGACTT (SEQ ID NO: 2) |

| 16S sequencing | |
|---|---|
| 515F | GTGCCAGCMGCCGCGGTAA (SEQ ID NO: 3) |
| 518F | CCAGCAGCCGCGGTAATACG (SEQ ID NO: 4) |

| RecA PCR | |
|---|---|
| recA_F | AGCTCGGTGGGGTTCTCTCATC (SEQ ID NO: 5) |
| recA_R | GCTTCCTCATACCACTGGTCATC (SEQ ID NO: 6) |

| RecA sequencing | |
|---|---|
| recA_F | GGTACCACTGCCATCTTCATTA (SEQ ID NO: 7) |
| 16S rRNA gene sequence | TCATTGGAGAGTTTGATCCTGGCTCAGGAC GAACGCTGGCGGCGTGCTTAACACATGCA AGTCGAACGGAAAGGCCCTGCTTTTGTGG GGTGCTCGAGTGGCGAACGGGTGAGTAAC ACGTGAGTAACCTGCCCTTGACTTTGGGAT AACTTCAGGAAACTGGGGCTAATACCGGAT AGGAGCTCCTGCTGCATGGTGGGGGTTGG AAAGTTTCGGCGGTTGGGGATGGACTCGC GGCTTATCAGCTTGTTGGTGGGGTAGTGG CTTACCAAGGCTTTGACGGGTAGCCGGCC TGAGAGGGTGACCGGCCACATTGGGACTG AGATACGGCCCAGACTCCTACGGGAGGCA GCAGTGGGGAATATTGCACAATGGGCGGA AGCCTGATGCAGCAACGCCGCGTGCGGGA TGACGGCCTTCGGGTTGTAAACCGCTTTCG CCTGTGACGAAGCGTGAGTGACGGTAATG GGTAAAGAAGCACCGGCTAACTACGTGCC AGCAGCCGCGGTGATACGTAGGGTGCGAG CGTTGTCCGGATTTATTGGGCGTAAAGGGC TCGTAGGTGGTTGATCGCGTCGGAAGTGT AATCTTGGGGCTTAACCCTGAGCGTGCTTT CGATACGGGTTGACTTGAGGAAGGTAGGG GAGAATGGAATTCCTGGTGGAGCGGTGGA ATGCGCAGATATCAGGAGGAACACCAGTG GCGAAGGCGGTTCTCTGGGCCTTTCCTGA CGCTGAGGAGCGAAAGCGTGGGGAGCGA ACAGGCTTAGATACCCTGGTAGTCCACGCT GTAAACGGTGGGTACTAGGTGTGGGGTCC ATTCCACGGGTTCCGCGCCGTAGCTAACG CTTTAAGTACCCCGCCTGGGGAGTACGGC CGCAAGGCTAAAACTCAAAGGAATTGACGG GGCCCCGCACAAGCGGCGGAGCATGCGG ATTAATTCGATGCAACGCGTAGAACCTTAC CTGGGTTTGACATGGATCGGGAGTGCTCA GAGATGGGTGTGCCTCTTTTGGGGTCGGT TCACAGGTGGTGCATGGCTGTCGTCAGCT CGTGTCGTGAGATGTTGGGTTAAGTCCCG CAACGAGCGCAACCCTTGTTCACTGTTGCC |
| | AGCACGTTATGGTGGGGACTCAGTGGAGA CCGCCGGGGTCAACTCGGAGGAAGGTGG GGATGACGTCAAGTCATCATGCCCCTTATG TCCAGGGCTTCACGCATGCTACAATGGCTG GTACAGAGAGTGGCGAGCCTGTGAGGGTG AGCGAATCTCGGAAAGCCGGTCTCAGTTC GGATTGGGGTCTGCAACTCGACCTCATGAA GTCGGAGTCGCTAGTAATCGCAGATCAGC AACGCTGCGGTGAATACGTTCCCGGGGCT TGTACACACCGCCCGTCAAGTCATGAAAGT TGGTAACACCCGAAGCCGGTGGCCTAACC GTTGTGGGGGAGCCGTCGAAGGTGGGACT GGTGATTAGGACTAAGTCGTAACAAGGTAG CCGTACCGGAAGGTGCGGCTGGATCACCT CCTTT (SEQ ID NO: 8) |

**[Table 2]**

| Classification and immunity traits of secured *C. acnes* strains | | | |
|---|---|---|---|
| Strains | Sequence type | Ribotype | Immunological phenotype |
| ATCC 6919 | Type I | RT1 | Invalid |
| CJIN-1-1 | | | |
| CJIN-1-2 | | | |
| CJRS-10651 | Type II | RT2 | Valid |
| CJRS-10652 | | | |
| CJRS-10653 | | | |
| CJRS-10654 | | | |
| CJRS-10655 | | | |
| CJRS-10656 | | | |
| ATCC 11828 | Type II | RT2 | Invalid |
| CJIN-2-1 | | | |
| CJIN-2-2 | | | |
| CJIN-3-1 | Type III | RT9 | Invalid |

The sequence type and ribotype of the secured *C. acnes* strains were classified according to the eMLST analysis result of the following Example 202, and the immunological phenotype was classified according to the immunity index analysis result of the following Example 3-3.

As shown in Table 2 above, it was confirmed that the 6 strains selected in the present application, CJRS-10651 strain, CJRS-10652 strain, CJRS-10653 strain, CJRS-10654 strain, CJRS-10655 strain, and CJRS-10656 strain had a characteristic in that the sequence type was Type II, and the ribotype was RT2, and the immunological phenotype was effective in common.

### Experimental example 2. Culture product acquisition

The strain shown in Table 2 above, and B. *fragilis* (*Bacteroides fragilis*, KCTC5013) strain, and *S*. *epidermidis* (*Staphylococcus epidermidis*, KCTC3958) strain used as a comparison group were cultured in an RCM broth (BD Difco) or a vegetable broth (the composition of the vegetable broth was shown in Table 3 below) for 48 hours, respectively, and then culture products (cell free supernatant) except for the strains were secured by centrifuging using a centrifuge (4000g, 20 minutes, room temperature).

**[Table 3]**

| Vegetable broth composition | | | |
|---|---|---|---|
| Component | Company | % (w/v) | Volume (g/L) |
| Glucose | Sigma aldrich | 0.5 | 5 |
| Peptone from vegetable | Sigma aldrich | 2.5 | 25 |
| Sodium chloride | Oxoid | 0.5 | 5 |
| Sodium acetate | Sigma aldrich | 0.3 | 3 |
| Cysteine HCl | Sigma aldrich | 0.05 | 0.5 |

The pH of the secured culture products was adjusted to pH 7 using NaOH, and then the culture products were filtered with a filter with a 0.22µm pore size, and the culture products filtered to 3kDa or less through a 3kDa amicon filter were obtained, and they were prepared as final test substances.

In the following examples, as substances to induce an inflammatory response, the culture products of the *C. acnes* (ATCC 6919) strain of the Table 2 were adjusted to pH 7, and then the culture products filtered with a 0.22µm pore size filter were prepared and used.

### Example 1. Hemolytic activity test of skin-derived strains

In order to confirm presence or absence of toxicity due to the hemolytic activity of the 6 skin-derived strains selected in the present application (CJRS-10651 strain, CJRS-10652 strain, CJRS-10653 strain, CJRS-10654 strain, CJRS-10655 strain, and CJRS-10656 strain), a hemolytic test was performed.

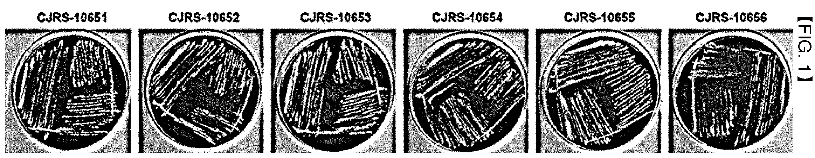

The skin-derived strains activated in an RCM broth were smeared on sheep blood agar, respectively, and cultured in an anaerobic chamber at 37°C for 48 hours. It was confirmed that a clear zone was produced as red blood cells were lysed around the colony cluster, and this was shown in FIG. 1.

As shown in FIG. 1, as the test result, the hemolytic activity was not shown for all the selected strains in the present application (CJRS-10651 strain, CJRS-10652 strain, CJRS-10653 strain, CJRS-10654 strain, CJRS-10655 strain, and CJRS-10656 strain), and thus, the strains selected in the present application was judged as safe.

### Example 2. Selected strain gene analysis

### Example 2-1. Confirmation of phylogenetic feature of strains.

*C. acnes* has been known to have a big difference in intraspecies level genome. As the difference in intraspecies level genome is actually big, it is currently classified as the same *C. acnes* species, but it is suggested that type I should be reclassified to *C*. *acnes* subspecies *acnes,* and type II should be reclassified to *C. acnes* subspecies *defendens*, and type III should be reclassified to *C. acnes* subspecies *elongatum.* These types have been known to have different 16S rDNA sequences, and be divided into various ribotypes (RT) by relative abundance of skin metagenome of several various people.

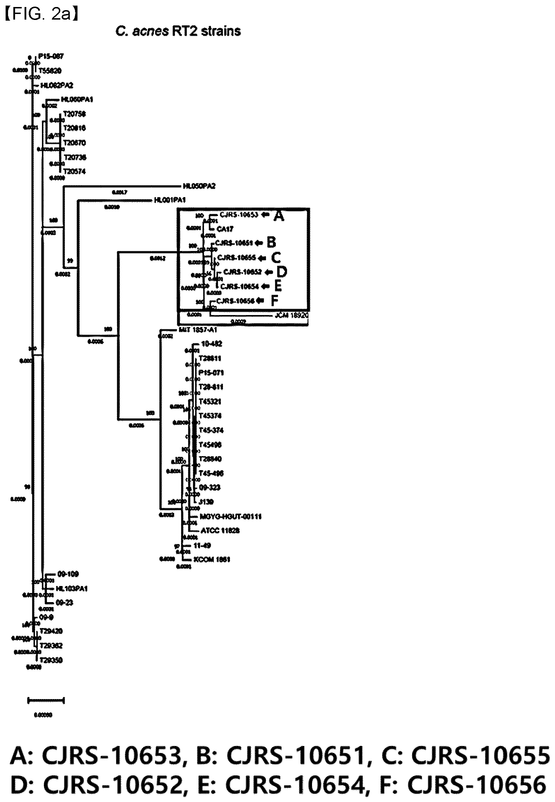

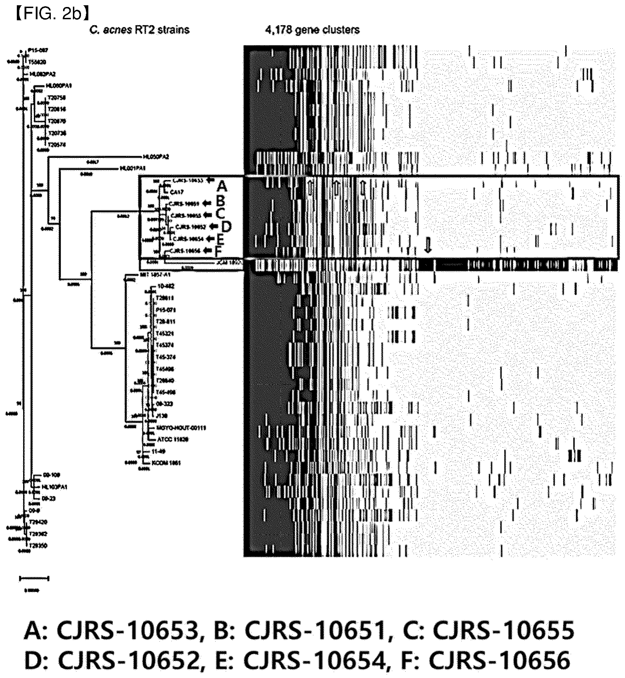

The ribotypes (RT) of the strains prepared in Experimental example 1 were confirmed through whole genome sequencing (WGS) of each strain (This result was shown in Table 2 above). Using whole genome data of the 6 strains, of which the ribotype was RT2, selected in the present application (CJRS-10651 strain, CJRS-10652 strain, CJRS-10653 strain, CJRS-10654 strain, CJRS-10655 strain, and CJRS-10656 strain), and the 37 strains, of which the ribotype was RT2, secured from NCBI genome site (reference strains), based on a rotary tool, a core gene and an accessory gene were obtained. After confirming the phylogenetic feature using sequences of the whole genome, alignment was conducted using MUSCLE algorithm, and a tree shape was represented by a maximum likelihood method, and this was shown in FIG. 2a and FIG. 2b. In other words, the phylogenetic tree was drawn using a core gene set, and it was shown as a drawing in a matrix form including the core gene and accessory gene set in FIG. 2a and FIG. 2b. The score at the edge is a confidence value obtained by repeating a bootstrap 10,000 times. The 6 strains selected in the present application were written in red in a red box in FIG. 2a, and indicated with a left arrow, and the reference strains were written in black.

In FIG. 2b, the gene clusters which the strains had were expressed in blue.

As could be confirmed in FIG. 2b, it was confirmed that the reference strains of which ribotype was RT2 (in FIG. 2a, other strains not included in the red box) had specific gene clusters, but the 6 strains isolated in the present application had no specific gene clusters as above (this part was indicated with an upward arrow in FIG. 2b).

In FIG. 2a, the JCM 18920 strain represented in black among the strains included in the red box, had an additional accessory gene compared to the 6 strains isolated in the present application, as could be confirmed in FIG. 2b. This point showed that the JCM 18920 strain had a different appearance from the 6 strains isolated in the present application, and it was confirmed that it was different from the 6 strains isolated in the present application (this part was indicated with a downward arrow in FIG. 2b).

### Example 2-2. eMLST (Expanded Multi-locus Sequence Typing) analysis of strains

Additionally, in order to compare the 37 strains, of which ribotype was RT2, secured from NCBI genome site (reference strains), and the 6 strains, of which ribotype was RT2, selected in the present application (CJRS-10651 strain, CJRS-10652 strain, CJRS-10653 strain, CJRS-10654 strain, CJRS-10655 strain, and CJRS-10656 strain), using eBURSTv3 program (http://eburst.mlst.net/default.asp), eMLST analysis was performed (Dreno et al. 2018). eMLST analysis is separating using sequences of genes which are conserved in *C. acnes* strains and improve phylogenetic resolution than 16S rRNA sequences. They were divided into specific sequence types (ST) depending on sequence variation of the genes, and divided STs were grouped by specific clonal complex (CC) as Table 4 below depending on frequency. The ST is a method of dividing based on diversity of sequences, and CC is a method of classifying the divided ST into one cluster using the difference in sequences. Table 4 below shows sequence type (ST) and clonal complex through eMLST analysis of the selected RT2 *C. acnes:*

**[Table 4]**

| Strains | *aroE* | *atpD* | *gmk* | *gua A* | *lepA* | *sod A* | *tly* | *camp 2* | ST | Clonal comple x (CC) |
|---|---|---|---|---|---|---|---|---|---|---|
| *C. acnes* strain KCOM 1861 (= ChDC B594) | 17 | 4 | 2 | 4 | 2 | 12 | 10 | 28 | 6 | CC6 (type II) |
| *C. acnes* HL082PA 2 | 17 | 4 | 2 | 4 | 2 | 3 | 10 | 10 | 6 | CC6 (type II) |
| *C. acnes* HL060PA 1 | 17 | 4 | 2 | 4 | 2 | 3 | 10 | 10 | 6 | CC6 (type II) |
| *C. acnes* strain T20574 | 17 | 4 | 2 | 4 | 2 | 3 | 10 | 10 | 6 | CC6 (type II) |
| *C. acnes* strain T20670 | 17 | 4 | 2 | 4 | 2 | 3 | 10 | 10 | 6 | CC6 (type II) |
| *C. acnes* strain T20736 | 17 | 4 | 2 | 4 | 2 | 3 | 10 | 10 | 6 | CC6 (type II) |
| *C. acnes* strain T20758 | 17 | 4 | 2 | 4 | 2 | 3 | 10 | 10 | 6 | CC6 (type II) |
| *C. acnes* strain T20816 | 17 | 4 | 2 | 4 | 2 | 3 | 10 | 10 | 6 | CC6 (type II) |
| *C. acnes* subsp. *defenden* s strain 09-109 | 17 | 9 | 2 | 4 | 2 | 3 | 10 | 10 | 25 | CC6 (type II) |
| *C. acnes* HL103PA 1 | 17 | 9 | 2 | 4 | 2 | 3 | 10 | 10 | 25 | CC6 (type II) |
| *C. acnes* HL050PA 2 | 17 | 4 | 2 | 17 | 2 | 3 | 11 | 11 | 26 | CC6 (type II) |
| *C. acnes* subsp. defenden s ATCC 11828 | 17 | 4 | 2 | 4 | 2 | 12 | 10 | 13 | 27 | Singleto n (type II) |
| *C. acnes* J139 | 17 | 4 | 2 | 16 | 2 | 12 | 10 | 12 | 27 | Singleto n (type II) |
| KCTC 3320 | 17 | 4 | 2 | 4 | 2 | 12 | 10 | 13 | 27 | Singleto n (type II) |
| *C. acnes* subsp. *defenden* s strain 09-323 | 17 | 4 | 2 | 16 | 2 | 12 | 10 | 12 | 28 | CC30 (previou sly CC72) (type II) |
| *C. acnes* HL001 PA 1 | 17 | 4 | 2 | 4 | 2 | 6 | 10 | 12 | 30 | CC30 (previou sly CC72) (type II) |
| *C. acnes* strain 09-9 | 17 | 4 | 2 | 4 | 2 | 3 | 10 | 19 | 65 | CC6 (type II) |
| *C. acnes* strain T29350 | 17 | 4 | 2 | 4 | 2 | 3 | 10 | 19 | 65 | CC6 (type II) |
| *C. acnes* strain T29362 | 17 | 4 | 2 | 4 | 2 | 3 | 10 | 19 | 65 | CC6 (type II) |
| *C. acnes* strain T29420 | 17 | 4 | 2 | 4 | 2 | 3 | 10 | 19 | 65 | CC6 (type II) |
| **CJRS-10651** | **17** | **4** | **2** | **4** | **4** | **6** | **10** | **12** | **69** | **CC72 (type II)** |
| **CJ RS-10652** | **17** | **4** | **2** | **4** | **4** | **6** | **10** | **12** | **69** | **CC72 (type II)** |
| **CJRS-10654** | **17** | **4** | **2** | **4** | **4** | **6** | **10** | **12** | **69** | **CC72 (type II)** |
| **CJRS-10655** | **17** | **4** | **2** | **4** | **4** | **6** | **10** | **12** | **69** | **CC72 (type II)** |
| **CJRS-10656** | **17** | **4** | **2** | **4** | **4** | **6** | **10** | **12** | **69** | **CC72 (type II)** |
| *C. acnes* isolate MGYG-HGUT-00111 | 17 | 4 | 2 | 4 | 2 | 12 | 10 | 12 | 72 | CC72 (type II) |
| *C. acnes* subsp. *defenden* s strain 11-49 | 17 | 4 | 2 | 4 | 2 | 12 | 10 | 12 | 72 | CC72 (type II) |
| *C. acnes* subsp. *defenden* s strain 10-482 | 17 | 4 | 14 | 16 | 2 | 12 | 10 | 39 | 130 | Singleto n (type II) |
| *C. acnes* strain MIT 1857-A1 | 20 | 4 | 2 | 4 | 2 | 6 | 10 | 42 | 137 | CC72 (type II) |
| *C. acnes* strain P15-071 | 17 | 4 | 2 | 16 | 2 | 20 | 10 | 12 | 139 | CC72 (type II) |
| *C. acnes* strain T45496 | 17 | 4 | 2 | 16 | 2 | 20 | 10 | 12 | 139 | CC72 (type II) |
| *C. acnes* strain T45374 | 17 | 4 | 2 | 16 | 2 | 20 | 10 | 12 | 139 | CC72 (type II) |
| *C. acnes* strain T28840 | 17 | 4 | 2 | 16 | 2 | 20 | 10 | 12 | 139 | CC72 (type II) |
| *C. acnes* strain T28811 | 17 | 4 | 2 | 16 | 2 | 20 | 10 | 12 | 139 | CC72 (type II) |
| *C. acnes* strain T45321 | 17 | 4 | 2 | 16 | 2 | 20 | 10 | 12 | 139 | CC72 (type II) |
| *C. acnes* strain T45-496 | 17 | 4 | 2 | 16 | 2 | 20 | 10 | 12 | 139 | CC72 (type II) |
| *C. acnes* strain T45-374 | 17 | 4 | 2 | 16 | 2 | 20 | 10 | 12 | 139 | CC72 (type II) |
| *C. acnes* strain T28-811 | 17 | 4 | 2 | 16 | 2 | 20 | 10 | 12 | 139 | CC72 (type II) |
| *C. acnes* strain T55820 | 17 | 4 | 2 | 4 | 2 | 3 | 36 | 10 | 150 | CC6 (type II) |
| *C. acnes* strain P15-087 | 17 | 4 | 2 | 4 | 2 | 3 | 36 | 10 | 150 | CC6 (type II) |
| *C. acnes* strain CA17 | 17 | 4 | 2 | 4 | 4 | 6 | 10 | 46 | 153 | NA |
| **CJRS-10653** | **17** | **4** | **2** | **4** | **4** | **6** | **10** | **46** | **153** | **NA** |
| *C. acnes* strain 09-23 | 17 | 9 | 2 | 4 | 2 | 3 | 32 | 10 | 25 or 128 | CC6 (type II) |
| *C. acnes* JCM 18920 | 17 | - | 2 | 4 | - | 6 | 10 | - | NA | NA |

As could be confirmed in Table 4 above, in the 6 strains selected in the present application, as the result of the eMLST analysis, it was confirmed that the ST (sequence type) was 69 or 153 (in case of CJRS-10651 strain, CJRS-10652 strain, CJRS-10654 strain, CJRS-10655 strain, and CJRS-10656 strain, ST was 69, and CC (clonal complex) was CC72 (Type II), and in case of CJRS-10653 strain, ST was 153, and CC was NA (not applicable)).

It is confirmed that the difference between ST 69 and ST 153 is a single nucleotide sequence difference of camp2which is a hemolytic factor-related gene, and the difference is weak, so it can be judged that the CC of the CJRS-10653 strain confirmed as ST 153 is close to CC72 (Type II) which is the CC of the CJRS-10651 strain, CJRS-10652 strain, CJRS-10654 strain, CJRS-10655 strain, and CJRS-10656 strain.

As the result of the eMLST analysis, *C. acnes* JCM 18920 and *C. acnes* CA17 strains were confirmed as strains having similar eMLST distribution to the 6 strains selected in the present application (CJRS-10651 strain, CJRS-10652 strain, CJRS-10653 strain, CJRS-10654 strain, CJRS-10655 strain, and CJRS-10656 strain), but due to the following reasons, the 6 strains selected in the present application was confirmed as different from the *C. acnes* JCM 18920 strain and *C. acnes* CA17 strain.

As the *C. acnes* JCM 18920 strain had an additional accessory gene differently from the 6 strains selected in the present application as confirmed in Example 2-1 above, it was confirmed that it was different from the 6 strains selected in the present application.

In order to confirm that the 6 strains selected in the present application was different from the *C. acnes* CA17 strain, genome comparison of the *C. acnes* CA17 strain was conducted. As a result of confirming through the WGS and genome sequence alignment results of the 6 strains selected in the present application and the *C. acnes* CA17 strain, it was confirmed that the plasmid of 1-3467 regions in the 4th scaffold was present specifically only to the *C. acnes* CA17 strain. As a result of matching the regions with nucleotide sequence database (GenBank, EMBL, DDBJ, PDB, and RefSeq) by BLAST, the regions were confirmed as plasmid sequences derived from the *Salmonella enterica* strain. In other words, the *C. acnes* CA17 strain had a plasmid derived from the *Salmonella enterica* strain, so it was confirmed that it was different from the 6 strains selected in the present application.

### Example 2-3. Orthologous Gene analysis of strains

Orthologous genes of the 6 strains, of which ribotype was RT2, selected in the present application (CJRS-10651 strain, CJRS-10652 strain, CJRS-10653 strain, CJRS-10654 strain, CJRS-10655 strain, and CJRS-10656 strain) were analyzed through an orthofinder.

Genes present strain-specifically were selected through specific thresholds (identity > 40 % and coverage > 80 %) using BLASTP, and genes that were poorly annotated as hypothetical protein or specific family protein were excluded. In addition, strain-specific literature investigation was conducted, and genes mentioned in the literature related to *C. acnes* and immunophenotypes were organized into categories. Some of these results were shown in Table 5 below.

**[Table 5]**

| Unique genetic organization of selected strains | | | | |
|---|---|---|---|---|
| Gene product | Function | Accession No. | Selected^{‡} | ATCC 11828 |
| Lrp/AsnC family transcriptional regulator | Branched amino acid metabolic pathway regulator | WP_002531510. 1 | + | - |
| Lactococcin 972 family | Bacteriocin | WP_070651130.1 | + | - |
| bacteriocin | | | | |
| Beta-glucuronidase | Bacterial oncogene | WP_002518535. 1 | - | + |
| 2-isopropylmalate synthase | L-Leucine metabolic pathway | WP_142263178. 1 | - | + |
| 3-isopropylmalate dehydratase | | WP_002513330. 1 | | |
| Type I-E CRISPR-associated protein Cse1/CasA | CRISPR adaptive immune system | WP_002514606. 1 | - | + |
| Type I-E CRISPR-associated protein Cas7/Cse4/CasC | | WP_002514608. 1 | | |
| CRISPR-associated helicase/endonuclease Cas3 | | WP_002514605. 1 | | |

(Selected^{‡}: 6 strains, of which ribotype was RT2, selected in the present application (CJRS-10651 strain, CJRS-10652 strain, CJRS-10653 strain, CJRS-10654 strain, CJRS-10655 strain, and CJRS-10656 strain); ATCC 11828: conventional strain of which ribotype was RT2 (reference))

As shown in Table 5 above, it was confirmed that the 6 strains, of which ribotype was RT2, selected in the present application (CJRS-10651 strain, CJRS-10652 strain, CJRS-10653 strain, CJRS-10654 strain, CJRS-10655 strain, and CJRS-10656 strain) were different from the strain conventionally discovered, of which ribotype was RT2 (control group, ATCC 11828 strain).

In other words, it was confirmed that the 6 strains of which ribotype was RT2, selected in the present application, differently from the ATCC 11828 strain, comprised an Lrp/AsnC family transcriptional regulator (NCBI Reference Sequence: WP_002531510.1) or a gene encoding the same, and a lactococcin 972 family bacteriocin (NCBI Reference Sequence: WP_070651130.1) or a gene encoding the same.

In addition, it was confirmed that the 6 strains, of which ribotype was RT2, selected in the present application did not comprise a beta-glucuronidase (NCBI Reference Sequence: WP_002518535.1) or a gene encoding the same, differently from the ATCC 11828 strain. The beta-glucuronidase has been well known to make calculus by aggregating bile derived from gall bladder, and known to cause colon cancer.

In addition, it was confirmed that the 6 strains, of which ribotype was RT2, selected in the present application did not comprise 2-isopropylmalate synthase (NCBI Reference Sequence: WP_142263178.1) or a gene encoding the same, and 3-isopropylmalate dehydratase (NCBI Reference Sequence: WP_002513330.1) or a gene encoding the same (L-Leucine pathway was deleted), differently from the ATCC 11828 strain.

In addition, it was confirmed that the 6 strains, of which ribotype was RT2, selected in the present application did not comprise type I-E CRISPR-associated protein Cse1/CasA (NCBI Reference Sequence: WP_002514606.1) or a gene encoding the same, type I-E CRISPR-associated protein Cas7/Cse4/CasC (NCBI Reference Sequence: WP_002514608.1) or a gene encoding the same, and CRISPR-associated helicase/endonuclease Cas3 (NCBI Reference Sequence: WP_002514605.1) or a gene encoding the same, differently from the ATCC 11828 strain.

### Example 3. Immunity index analysis

The immunity indexes of immunocytes (Raw264.7 macrophages) for the culture products of the 6 strains selected in the present application (CJRS-10651 strain, CJRS-10652 strain, CJRS-10653 strain, CJRS-10654 strain, CJRS-10655 strain, or CJRS-10656 strain) were confirmed.

### Example 3-1. Immunocyte culture

As immunocytes used in the present experiment, Raw264.7 (KCLB 40071) macrophages were used. One week before entering the present experiment, the Raw264.7 macrophages were cultured and maintained in FBS 10%(v/v) DMEM. Before 18 hours prior to the experiment, the Raw264.7 macrophages were seeded in a flat bottom 96 well plate by 5 × 10⁴/well.

### Example 3-2. Co-culture of strain culture products and immunocytes:

In order to induce an inflammatory response in the Raw264.7 macrophages, the culture products of the pathogen C. acnes (ATCC 6919) strain prepared in Experimental example 2 were treated to the Raw264.7 macrophages at 5µl/well, and RPMI Media (gibco) 95ul was added, and a total of 100µl was treated in a 37°C incubator for 1 hour. When the inflammatory response was induced, the culture products of the pathogen C. acnes (ATCC 6919) strain were treated as 5µl/well to all the groups except for a normal cell (leftmost of FIG. 3, Cell only) group to give an inflammatory stimulus.

After 1 hour, by adding a total of 100µl in a plate in which the culture products of the pathogen C. acnes (ATCC 6919) strain, which is an inflammation-inducing substance, in addition to the culture products of the strain prepared in Experimental example 2 (20µl) and fresh RPMI Media (80µl) (200µl/well in total), they were cultured in a 37°C incubator for a total of 24 hours.

In other words, in order to induce an immune response to the Raw264.7 macrophages, the culture products of the *C. acnes* (ATCC 6919) strain were treated, and after that, the culture products of the various strains were treated and immunity indexers were confirmed, and the result was shown in FIG. 3 and FIG. 4.

As a positive control group (Positive control), dexamethasone (sigma) (100nM) (Dex of FIG. 3) was used. As a negative control group (negative control), a normal cell in which a vegetable empty broth (Broth of FIG. 3) or fresh RPMI Media (gibco) was added (Cell only) was used.

### Example 3-3. Immunity index analysis

In order to confirm the viability and proliferation level of the Raw264.7 macrophages remained after securing all the supernatant (cultured supernatant) after 24 hours, cell counting kit 8 (Enzo, 5µl/well) + fresh RPMI media (95µl) were treated to react in a 37°C incubator for 2 hours, and then the absorbance was measured at 450nm.

Using the secured supernatant, the amounts of the inflammatory cytokines (IL-6 (interleukin 6), CCL2 (C-C Motif Chemokine Ligand 2), TNF-α (tumor necrosis factor alpha)) secreted from the Raw264.7 macrophages were measured by ELISA. The degree of reduction in the inflammatory cytokine level and inhibition of proliferation of the Raw264.7 macrophages were confirmed by type of the strains using the Raw264.7 macrophages, and it was shown in FIG. 3 and FIG. 4, and depending on the degree of the inflammatory inhibition function, they were marked as effective or non-effective strains (effective/non-effective results were shown in Table 2 above).

As shown in the left graph of FIG. 3 and FIG. 4, the normal cell (nothing was treated to the Raw264.7 macrophages; Cell only) did not secret the inflammatory cytokines, but when the culture products of the pathogen *C. acnes* (ATCC 6919) strain were treated to the Raw264.7 macrophages to induce an inflammatory response (Cell only w/ stimuli), the inflammatory cytokines (IL-6, CCL2, TNF) were significantly increased. As a positive control group, when an inflammatory response was induced with the culture products of the pathogen *C. acnes* (ATCC 6919) strain on the Raw264.7 macrophages and dexamethasone (Dex) was treated, it was confirmed that the amount of the inflammatory cytokines was reduced. As a negative control group, when an inflammatory response was induced with the culture products of the pathogen *C. acnes* (ATCC 6919) strain on the Raw264.7 macrophages and a vegetable empty broth was treated, the inflammatory response was not reduced.

When the culture products of the 6 strains selected in the present application (CJRS-10651 strain, CJRS-10652 strain, CJRS-10653 strain, CJRS-10654 strain, CJRS-10655 strain, or CJRS-10656 strain) were treated, respectively, an inflammatory response was induced with the culture products of the pathogen *C. acnes* (ATCC 6919) strain and the secreted amount of the inflammatory cytokines (IL-6, CCL2, TNF) was significantly reduced, and it was confirmed that such a result reduced the secreted amount of the inflammatory cytokines equivalently or more to the effect when the positive control group, dexamethasone (Dex) was used.

When the culture products of other types of *Cutibacterium* sp. Strain (ATCC6919, ATCC11828, CJIN1-1, CJIN1-2, CJIN2-1, CJIN2-2, CJIN3-1) or other bacteria (*B*. *fragilis* or *S*. *epidermidis*) were treated, contrarily to the case of treating the culture products of the 6 strains selected in the present application, respectively, it was confirmed that there was no change in the secreted amount of the inflammatory cytokines (IL-6, CCL2, TNF) increased by inducing an inflammatory response with the culture products of the pathogen *C. acnes* (ATCC 6919) strain, or rather, it increased further significantly.

The downward graph of FIG. 3 shows the proliferation level of the Raw264.7 macrophages causing an inflammatory response by %, based on the normal cell (nothing was treated to the Raw264.7 macrophages; Cell only). As shown in the downward graph of FIG. 3, when an inflammatory response was induced by treating the culture products of the pathogen *C. acnes* (ATCC 6919) strain to the Raw264.7 macrophages (Cell only w/ stimuli), the proliferation of the Raw264.7 macrophages causing an inflammatory response was increased.

When the culture products of the 6 strains selected in the present application (CJRS-10651 strain, CJRS-10652 strain, CJRS-10653 strain, CJRS-10654 strain, CJRS-10655 strain, or CJRS-10656 strain) were treated, respectively, it was confirmed that the proliferation of the Raw264.7 macrophages was reduced equivalently or less to the case in that nothing was treated to the Raw264.7 macrophages.

### Example 4. Acne-causing strain, C. acnes (ATCC 6919) growth inhibitory effects

The antibacterial activity of the culture products of the 6 strains selected in the present application (CJRS-10651 strain, CJRS-10652 strain, CJRS-10653 strain, CJRS-10654 strain, CJRS-10655 strain, or CJRS-10656 strain) against *C. acnes* (ATCC 6919) known as an acne causing bacterium was confirmed. As a positive control group, doxycycline (0.025µg/ml) was used.

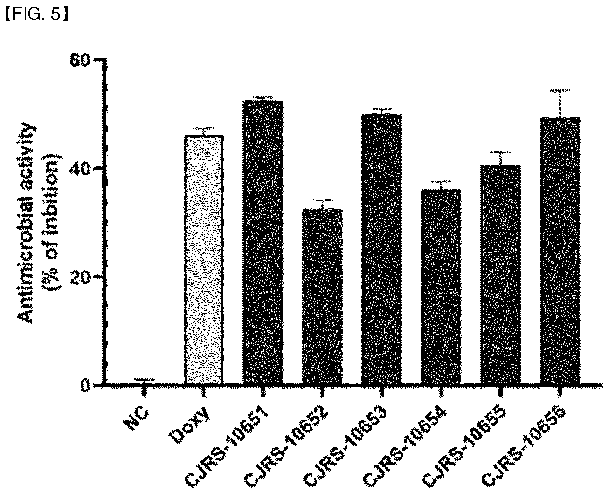

The concentrate of the culture products of the strain was diluted in an RCM broth, and added in a 96-well plate, and then the C. acnes ATCC 6919 strain was diluted to 10^6 CFU/mL using an RCM broth, and inoculated to the 96-well plate by 10%(v/v). After inoculating the strain, it was cultured in an anaerobic chamber under a 37°C environment for 48 hours, and then the absorbance was measured at 600 nm and the result was shown in FIG. 5.

FIG. 5 is a graph showing the growth inhibitory effect by % (Antimicrobial activity (% of inhibition)) based on the case in that nothing was treated to the ATCC 6919 strain (Negative Control; NC).

As shown in FIG. 5, it was confirmed that the growth of the *C. acnes* ATCC 6919 strain was reduced when doxycycline (Doxy) was treated as a positive control group. It was confirmed that even when the culture products of the 6 strains selected in the present application were treated, respectively, the growth of the *C. acnes* ATCC 6919 strain was reduced, and this result was equivalent or more to the effect when the positive control group, doxycycline (Doxy) was used.

### Example 5. Biofilm formation inhibition

It was confirmed that the culture products of the 6 strains selected in the present application (CJRS-10651 strain, CJRS-10652 strain, CJRS-10653 strain, CJRS-10654 strain, CJRS-10655 strain, or CJRS-10656 strain) inhibited biofilm formation of the S. *epidermidis* bacteria. As a positive control group, doxycycline (Doxy) (4µg/ml) was used.

After culturing the *S. epidermidis* bacteria in a 96-well cell culture plate at 1×10⁷CFU/mL for 24 hours and attaching them, nothing was treated (Negative Control; NC), or as a positive control group, doxycycline (Doxy) was treated, or the culture products of the 6 strains selected in the present application were treated, and they were cultured in a TSB (Tryptic Soy Broth, BD difco) broth under a condition of 37°C for 24 hours. After 24 hours, in order to measure the degree of biofilm formation, they were treated with Crystal violet (0.1% v/v) to stain the biofilm, and it was measured with Tecan at 570nm. After that, the biofilm formation inhibition rate was calculated by converting it by the formula of O.D. sample/O.D. control X 100.

Specifically, the *S*. *epidermidis* bacteria stored in a deep freezer were pulled out and inoculated in a TSB (Tryptic Soy Broth, BD difco) at 3%(v/v) and cultured at 37°C for 16 hours. The grown culture solution was diluted in a fresh TSB broth so as to be 1×10⁷CFU/mL. The freeze-dried culture solution which was an experimental group was diluted with distilled water according to the concentration to prepare samples.

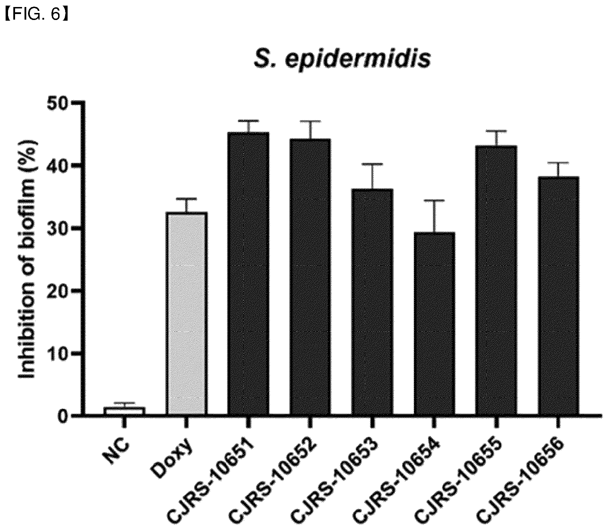

Each well was composed of a total of 200µl by treating the culture products 5%(v/v) 10µl of the *S. epidermidis* bacteria forming a biofilm of 100µl in each well of a 96-well microtiter polystyrene plate and the 6 strains selected in the present application (CJRS-10651 strain, CJRS-10652 strain, CJRS-10653 strain, CJRS-10654 strain, CJRS-10655 strain, or CJRS-10656 strain), and the TSB broth 90ul together. They were cultured in a plate state in a 37°C incubator for 24 hours. The microbial strains cultured for 24 hours were under a pretreatment process for staining with crystal violet solution. The supernatant in which the biomembrane at the base side of the strain plate was excluded was removed by suction. Each well was washed once using 1XPBS solution of 100µl. The washed biomembrane was stained under a condition of room temperature without light for 20 minutes using 0.1% crystal violet solution. After completing the staining, it was washed using 1XPBS solution 100ul. The stained biomembrane was torn off from the plate using 33% acetic acid solution. For the lysed biomembrane, formation/inhibition was evaluated through a value at a wavelength of OD570nm using an optical density plate reader device, and this result was shown in FIG. 6.

As shown in FIG. 6, in case in that nothing was treated to the *S*. *epidermidis* bacteria (Negative Control; NC), the biofilm formation inhibitory effect was not shown. When doxycycline (Doxy) was treated to the *S. epidermidis* bacteria as the positive control group, the biofilm formation inhibitory effect was shown.

Also, when the culture products of the 6 strains selected in the present application (CJRS-10651 strain, CJRS-10652 strain, CJRS-10653 strain, CJRS-10654 strain, CJRS-10655 strain, or CJRS-10656 strain) were treated to the *S. epidermidis* bacteria, respectively, the biofilm formation inhibitory effect was shown, and this result confirmed that biofilm formation was inhibited equivalently or more to the effect when doxycycline (doxy) which was the positive control group was used.

### Example 6. Toxicity test

It was confirmed that the culture products of the 6 strains selected in the present application (CJRS-10651 strain, CJRS-10652 strain, CJRS-10653 strain, CJRS-10654 strain, CJRS-10655 strain, or CJRS-10656 strain) had no cytotoxicity.

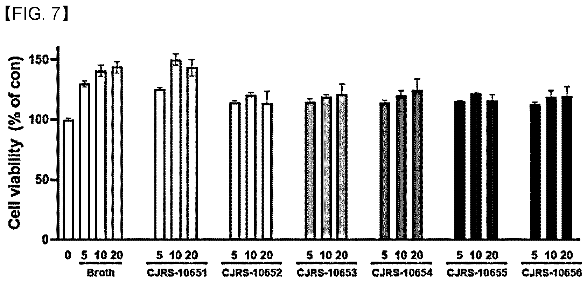

After attaching HaCaT cells to a 96-well cell culture plate at 2×10^4/well, respectively, for 24 hours, a broth (vegetable empty broth) was concentrated and added (0%(v/v), 5%(v/v), 10%(v/v), or 20%(v/v)) (Negative Control; Broth), or the culture products of the 6 strains selected in the present application were concentrated, respectively, and then added (5%(v/v), 10%(v/v), 20%(v/v)), and cultured under conditions of 5% CO2, 37°C for 24 hours. After 24 hours, the cultured cell broth was removed, and cell counting kit-8 solution was treated to the cells and they were reacted for 2 hours, and then the absorbance was measured at 450nm with Tecan. After that, by converting with the formula of O.D sample /O.D control X 100, the survival rate (Cell viability) of the HaCaT cell line was calculated and the result was shown in FIG. 7.

As shown in FIG. 7, it was confirmed that compared to the case in that nothing was treated to the HaCaT cells, also in the case in that the culture products of the 6 strains selected in the present application (CJRS-10651 strain, CJRS-10652 strain, CJRS-10653 strain, CJRS-10654 strain, CJRS-10655 strain, or CJRS-10656 strain) were treated, respectively, they did not affect the survival rate of the HaCaT cells and rather increased the cell survival rate.

This result confirmed that the culture products of the 6 strains selected in the present application had no cytotoxicity, and rather, an effect of reducing cytotoxicity could be expected.

### Example 7. Skin efficacy test

The skin regeneration efficacy of the culture products of the 6 strains selected in the present application (CJRS-10651 strain, CJRS-10652 strain, CJRS-10653 strain, CJRS-10654 strain, CJRS-10655 strain, or CJRS-10656 strain) was confirmed.

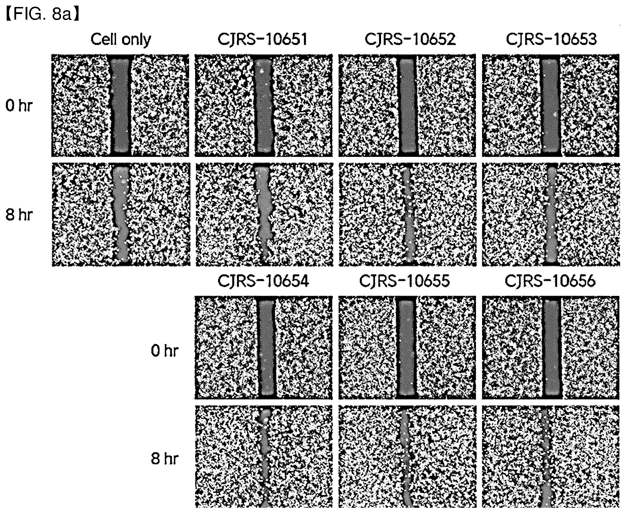

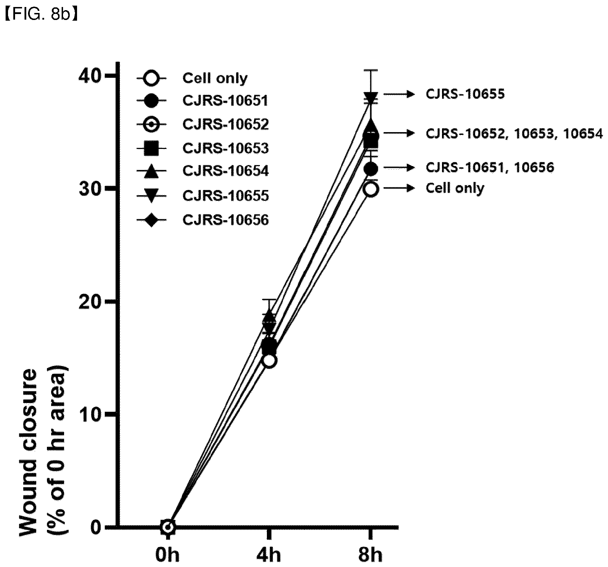

The HaCaT cells were seeded to Cell culture insert (Ibidi) in an amount of 1×10^6/ml by 70µl, respectively, and then cultured under conditions of 5% CO2, 37°C for 24 hours. After 24 hours of the cell culture, the insert was removed using a sterilized forcep to make a wound condition required for wound healing assay and then wash with PBS twice. It was replaced with a broth comprising no FBS, and nothing was treated (Cell only), or the culture products of the 6 strains selected in the present application (CJRS-10651 strain, CJRS-10652 strain, CJRS-10653 strain, CJRS-10654 strain, CJRS-10655 strain, or CJRS-10656 strain) were added by 15%(v/v), respectively, and they were cultured under conditions of 5% CO2, 37°C. In 0 hour, 4 hours, and 8 hours after treating the culture products, cell photographs were taken using a microscope and the wound area was calculated using a cell profiler program, and converted by the formula of (0-hour area - n-hour area)/0-hour area x 100 to confirm the wound closure of the HaCaT cell line, and the results were shown in FIG. 8a and FIG. 8b.

As shown in FIG. 8a and FIG. 8b, compared to the case in that nothing was treated to the HaCaT cells (Cell only), when the culture products of the 6 strains selected in the present application (CJRS-10651 strain, CJRS-10652 strain, CJRS-10653 strain, CJRS-10654 strain, CJRS-10655 strain, or CJRS-10656 strain) were treated, respectively, the wound closure increased, and thereby, it was confirmed that the skin cell regeneration efficacy was excellent.

In addition, by adding the culture products of the 6strains selected in the present application to the HaCaT cell line, skin regeneration-related markers, Src and MMP2 (metalloproteinase-2) expression was confirmed. An increase in activity of Src means promoting regeneration of epidermal epithelial cells, dermal layers and vascular endothelia, and MMP2 is known to play a role in helping regeneration of extracellular matrix important in tissue regeneration and promoting movement of cells important in re-epithelialization such as keratinocytes and fibroblasts.

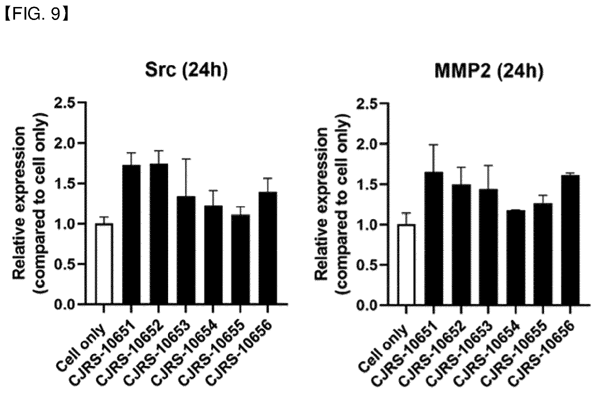

The HaCaT cells were seeded to a 24 well cell culture plate in an amount of 1×10^5, and then they were cultured under conditions of 5% CO2, 37 °C for 24 hours. After 24 hours of cell culture, it was replaced with a broth comprising no FBS, and nothing was treated (Cell only), or the culture products of the 6 strains selected in the present application (CJRS-10651 strain, CJRS-10652 strain, CJRS-10653 strain, CJRS-10654 strain, CJRS-10655 strain, or CJRS-10656 strain) were added by 15%(v/v), respectively, and they were reacted under conditions of 5% CO2, 37°C for 24 hours, and the expression of Src and MMP2 was confirmed on RNA, and the result was shown in FIG. 9.

As shown in FIG. 9, it was confirmed that the expression level of Src and MMP2 increased, when the culture products of the 6 strains selected in the present application (CJRS-10651 strain, CJRS-10652 strain, CJRS-10653 strain, CJRS-10654 strain, CJRS-10655 strain, or CJRS-10656 strain), compared to the case in that nothing was treated to the HaCaT cells (Cell only). This result shows the skin regeneration efficacy of the culture products of the 6 strains selected in the present application.

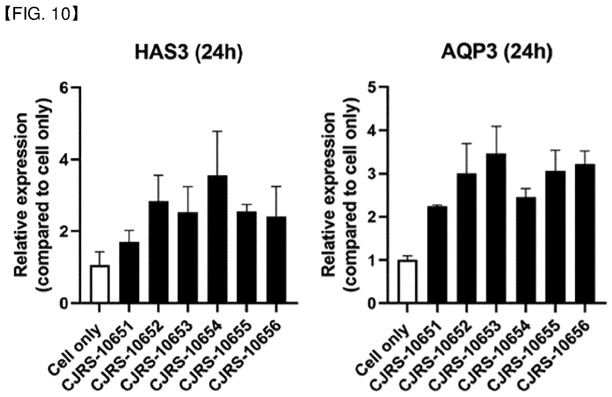

In addition, in order to confirm the skin moisturizing effect, the HaCaT cells were seeded to a 24 well cell culture plate in an amount of 1×10^5, and then cultured under conditions of 5% CO2, 37°C for 24 hours. After 24 hours of cell culture, it was replaced with a broth comprising no FBS, and nothing was treated (Cell only), or the culture products of the 6 strains selected in the present application (CJRS-10651 strain, CJRS-10652 strain, CJRS-10653 strain, CJRS-10654 strain, CJRS-10655 strain, or CJRS-10656 strain) were added by 15%(v/v), respectively, and they were reacted under conditions of 5% CO2, 37°C for 24 hours, and the expression of skin moisturizing function markers, HAS3 (Hyaluronic acid Synthase 3) and AQP3 (Aquaporin 3) was confirmed on RNA, and the result was shown in FIG. 10.

As shown in FIG. 10, it was confirmed that the expression level of HAS3 and AQP3 increased when the culture products of the 6 strains selected in the present application (CJRS-10651 strain, CJRS-10652 strain, CJRS-10653 strain, CJRS-10654 strain, CJRS-10655 strain, or CJRS-10656 strain) were treated, respectively, compared to the case in that nothing was treated to the HaCaT cells. This result shows that the skin moisturizing function strengthening efficacy of the culture products of the 6 strains selected in the present application.

### Example 8. Evaluation test of inflammation regulation effectiveness of living cells

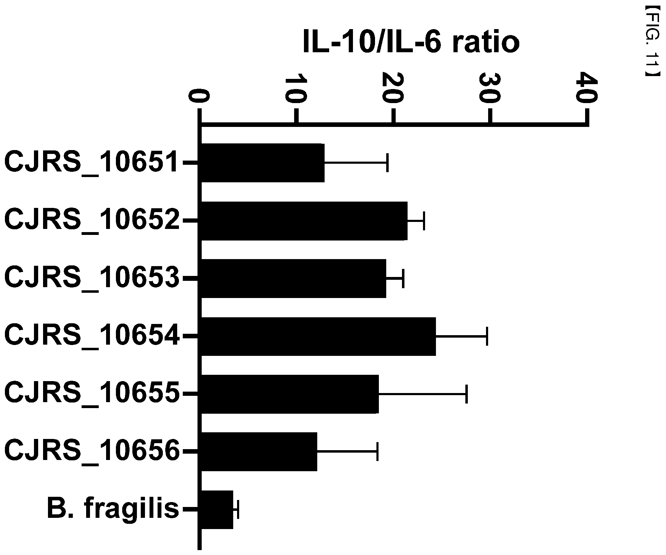

By culturing the 6 strains selected in the present application (CJRS-10651 strain, CJRS-10652 strain, CJRS-10653 strain, CJRS-10654 strain, CJRS-10655 strain, or CJRS-10656 strain) in a vegetable broth (Table 3) and then centrifuging (4000g, 20 minutes, room temperature), living cells except for the strain culture products were secured. The secured living cells and immunocytes (Raw264.7 macrophages) were co-cultured at the same ratio (1:1) of 5×10^4 / well, respectively, in a 96 well plate for 24 hours, and then the inflammation regulation efficacy of the strains was confirmed. As a control group, a *B*. *fragilis* strain known as an inflammation alleviating strain was used. The secreted amounts of the inflammatory cytokine (IL-6) and anti-inflammatory cytokine (IL-10) secreted from the macrophages were measured by ELISA, and then values thereof were calculated at a ratio of IL-10/IL-6 and they were shown in FIG. 11.

As could be confirmed in FIG. 11, the 6 strains selected in the present application (CJRS-10651 strain, CJRS-10652 strain, CJRS-10653 strain, CJRS-10654 strain, CJRS-10655 strain, or CJRS-10656 strain) reduced the secreted amount of the inflammatory cytokine (IL-6) and promoted the secreted amount of the anti-inflammatory cytokine (IL-10) compared to the *B*. *fragilis* strain, and therefore, overall, the anti-inflammatory activity was excellent.

### Example 9. Primary immunocyte culture and immunity index analysis

Immunity indexes of immunocytes were confirmed when the culture products of the 6 strains selected in the present application (CJRS-10651 strain, CJRS-10652 strain, CJRS-10653 strain, CJRS-10654 strain, CJRS-10655 strain, or CJRS-10656 strain) were treated.

The immunocytes used in the present experiment were mouse-derived marrow derived macrophages (mouse BMDM, Hanlim, Co., Ltd) and human monocyte derived macrophage (HMDM, ATCC). One week before entering the experiment, the cells were cultured under the following conditions. The cells were cultured under conditions of BMDM with 10ng/ml of mouse M-CSF or HMDM with 50ng/ml of human M-CSF in 10% RPMI Media, and fresh RPMI Media containing M-CSF were replaced at intervals of 3 days. Before 18 hours of the experiment, the immunocytes to be tested were seeded in a flat bottom 96 well plate at 5 × 10⁴/well.

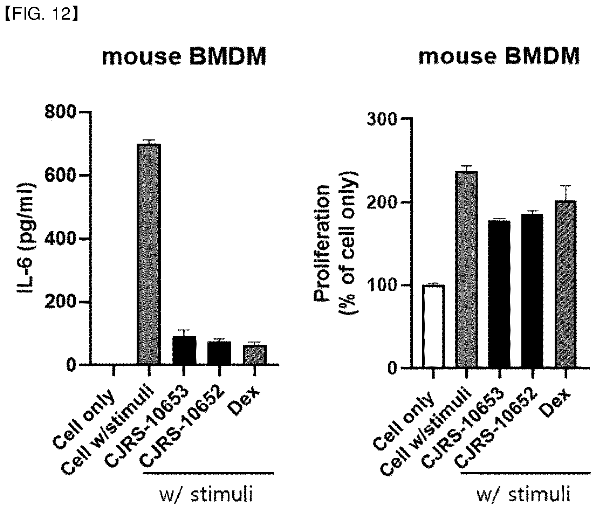

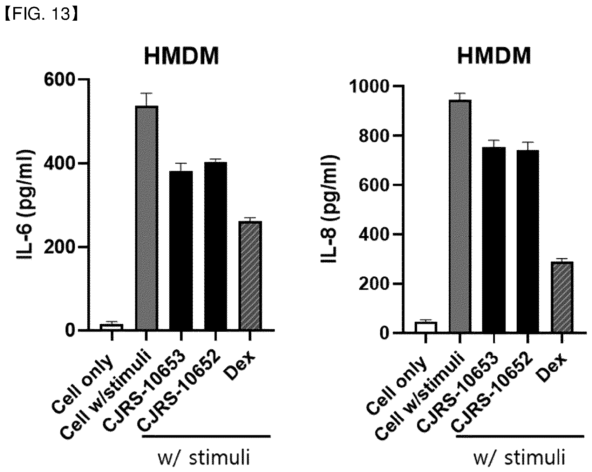

Mouse BMDM (FIG. 12) or HMDM (FIG. 13) were seeded at 5x10^4/well in a 96 well cell culture plate using a broth comprising M-CSF, and then cultured under conditions of 5% CO2, 37°C for 24 hours. During the present experiment, in order to induce an inflammatory response, the culture products of the *C. acnes* (ATCC 6919) strain were treated at 5µl/well, and RPMI Media 95µl was added, and total 100µl was treated in a 37°C incubator for 1 hour. During induction of the inflammatory response, in all the groups except for the normal cell (Cell only) group, the culture products of the *C. acnes* (ATCC 6919) strain were treated to give an inflammatory stimulus. After 1 hour, a total of 100µl, in which each of the culture products (20µl) of the CJRS-10652 strain or CJRS-10653 strain selected in the present application, respectively, and fresh Media (80µl) were added, was added in a plate in which an inflammation inducing substance was treated (total 200µl/well), and they were cultured in a 37°C incubator for 24 hours. As a positive control group (Positive control), dexamethasone (Dex) (100nM) was used. As a negative control group (negative control), normal cells (Cell only) treated with fresh Media were used. After culturing for 24 hours, all the cultured supernatant was secured, and in order to confirm the level of proliferation of the remaining immunocytes, cell counting kit-8 (5µl well) + fresh media (95µl) were treated and reacted in a 37°C incubator for 2 hours, and then the absorbance was measured at 450nm, and based on Cell only, the cell proliferation level was marked by %. Using the secured cell supernatant, the amounts of the cytokines (IL-6, IL-8) secreted from the immunocytes were measured by ELISA.

As could be confirmed in FIG. 12, when nothing was treated to the mouse BMDM cells (Cell only), the inflammatory cytokine (IL-6) was not secreted. In case of Cell w/ stimuli, secretion of the inflammatory cytokine (IL-6) increased significantly, and the proliferation of the mouse BMDM cells, which are immunocytes, increased significantly compared to the case in that nothing was treated (Cell only). It was confirmed that the increased amount of IL-6 and increased proliferation of the mouse BMDM cells were significantly lowered as an inflammatory response was induced, when dexamethasone (Dex) was used as a positive control group.

It was confirmed that the increased amount of IL-6 and increased proliferation of the mouse BMDM cells were significantly lowered as an inflammatory response was induced to the mouse BMDM cells, when the culture products of the CJRS-10652 strain or CJRS-10653 strain selected in the present application were treated, respectively, and it was confirmed that this result showed an equivalent or more excellent effect to the case in which dexamethasone, which was a positive control group, was used.

FIG. 13 is the result of confirming the secreted amounts of the inflammatory cytokines (IL-6, IL-8) using the HMDM cells instead of the mouse BMDM cells, and when nothing was treated to the HMDM cells (Cell only), the inflammatory cytokines (IL-6, IL-8) were hardly secreted. In case of Cell w/ stimuli, secretion of the inflammatory cytokines (IL-6, IL-8) increased significantly, but when dexamethasone (Dex) was used as a positive control group, an inflammatory response was induced to the HMDM cells and thus the increased amounts of the IL-6 and IL-8 were significantly reduced.

It was confirmed that the increased secreted amounts of IL-6 and IL-8 were significantly reduced as an inflammatory response was induced to the HMDM cells, even when the culture products of the strains selected in the present application (CJRS-10652 strain or CJRS-10653 strain) were treated, respectively.

### Example 10. Evaluation of rosacea animal model effectiveness

For the present animal effectiveness test, 32 5-week-old female BALB/c mice were purchased from Orient Bio company, and brought into a breeding room with a 12-hour artificial photoperiod, and acclimatized for one week, and then used in the experiment.

To make a rosacea animal model, hair on the mice's back area was removed using a depilator and hair removal cream. In 24 hours after depilation, to induce rosacea lesions, LL37 peptide (Peptron, Co., Ltd) of 320µM was under intradermal injection using an insulin syringe by 50µl. LL37 injection was performed twice a day, for 2 days (total 4 times), and the culture products of the strains selected in the present application (CJRS-10652 strain or CJRS-10653 strain) were intraperitoneally administered by 150µl with the second LL37 injection once a day, for 3 days (total 3 times). Doxycycline, which is one of conventional rosacea therapies, was peritoneally administered by 1mg/kg once a day, for 3 days (total 3 times).

After 24 hours of the last strain culture product administration, the size (area) of erythema was measured using a caliper, and the animals were euthanized using CO2 gas, and then the lesion area was taken as a photograph and the degree of the erythema was observed by naked eyes and the tissue photographs and the area of the inflammation area were shown in FIG. 14a and FIG. 14b as graphs.

As shown in FIG. 14a and FIG. 14b, it was confirmed that the erythema was largely shown on the skin when rosacea was induced by treating the LL37 peptide to the mice, compared to the case in which nothing was treated to the mice (Vehicle). Doxycycline (Doxy) was treated to the mice as a positive control group, and thereby, it was confirmed that the erythema was slightly reduced on the skin.

It was confirmed that the erythema was significantly reduced on the skin, when the culture products of the strains selected in the present application (CJRS-10652 strain or CJRS-10653 strain) were treated (peritoneally administered) to the mice, respectively, and this result confirmed that an extraordinarily excellent effect was shown even compared to the case in that doxycycline, which was the positive control group, was treated.

In addition, in order to investigate a pathological examination result of the injection animal model according to administration of the culture products of the strains selected in the present application (CJRS-10652 strain or CJRS-10653 strain), the skin of the LL37 peptide-administered area was extracted, and fixed in 4% formaldehyde solution for tissue staining and embedded in paraffin. After making fragments from the paraffin-embedded tissue, for observation of histological changes, hematoxylin-eosin (H&E) staining was conducted, and they were observed with a microscope, and tissue photographs were shown in FIG. 15, and histopathological evaluation scores were shown in Table 6 below.

**[Table 6]**

| | **Epithelial hyperplasia** | **Dermal inflammation** | **Total** |
|---|---|---|---|
| **Vehicle** | 0 | 0 | 0 |
| **LL37** | 2.75 | 2.5 | 5.25 |
| **CJRS-10653** | **1.6** | **1.8** | **3.4** |
| **CJRS-10652** | **1.8** | **1.4** | **3.2** |
| **Doxy** | 1.2 | 2.4 | 3.6 |

As shown in FIG. 15, compared to the case in that nothing was treated to the mice (Vehicle), in the case in that the LL37 peptide was treated to the mice to induce rosacea (LL37), the area of the inflammation area increased significantly. It was confirmed that the area of the inflammation area was slightly reduced when doxycycline (Doxy) was treated to the mice as a positive control group. It was confirmed that the area of the inflammation area was significantly reduced by treating the culture products of the strains selected in the present application (CJRS-10652 strain or CJRS-10653 strain) to the mice, respectively, and this result confirmed that an extraordinarily excellent effect was shown even compared to the case in that doxycycline, which was the positive control group, was treated.

In addition, as shown in Table 6 above, compared to the case in that nothing was treated to the mice (Vehicle), in the case in that the LL37 peptide was treated to the mice to induce rosacea (LL37), epithelial hyperplasia and dermal inflammation increased significantly. It was confirmed that epithelial hyperplasia was reduced, but dermal inflammation was not reduced, when doxycycline (Doxy) was treated to the mice as a positive control group.

However, it was confirmed that the increased epithelial hyperplasia and dermal inflammation were significantly reduced, when the culture products of the strains selected in the present application (CJRS-10652 strain or CJRS-10653 strain) were treated to the mice, respectively.

From these results, it was confirmed that all the erythema, epithelial hyperplasia and dermal inflammation were reduced, when the culture products of the strains selected in the present application were intraperitoneally treated to the mice.

### Example 11. Confirmation of skin barrier improvement and moisturizing

Major causes of skin wrinkles and aging include a collapsed barrier in skin dermal layers, moisture loss and reduced collagen production, and the like. In particular, collagen is a major protein composing skin dermis and plays a role of maintaining the skin structure and elasticity. Deficiency of collagen is one of causes of occurrence of skin wrinkles, and it is known to show a decrease in production and also increase decomposition as aging progresses to induce depression of skin dermal layers and produce wrinkles of skin. In addition, moisture loss slows skin regeneration and reduces elasticity and causes fine wrinkles, and promotes skin aging. Therefore, by confirming important indexes of skin barrier (Filaggrin) improvement, collagen (Procollagen type I) production level, and moisturizing (Aquaporin-3) improvement, the skin wrinkle improvement and anti-aging effects can be confirmed.

Inflammation was induced using poly I:C to T&R HuSkin, which is a 3D mini tissue (organoid) manufactured from T&R Biofab, Co., Ltd. After that, by applying the strain culture products selected in the present application, changes in skin improvement indexes (barrier improvement and moisturizing) were confirmed.

### Test method

1) T&R HuSkin is a full thickness skin model composed of a dermal layer and an epidermal layer, and was manufactured using 3DXPrinter bioprinter of T&R Biofab, Co., Ltd.
2) For manufacturing of the dermal layer, bioink, in which porcine skin-derived extracellular matrix-based hydrogel and human-derived fibroblasts (Lonza, Basel, Switzerland) were mixed at a concentration of 100×10^4 cells/ml, was printed, and exposed in a 37 °C incubator for 40 minutes to gelate them.
3) Then, human-derived keratinocytes (Lonza) were aliquoted on the gelated dermal layer at 55×10^4 cells/insert, and cultured in a state completely sunk in keratinocytes growth media (KGM; Lonza) for 1 day.
4) One day later, in order to provide skin-like culture conditions, Air-liquid interface (ALI) conditions were implemented to expose only the surface of the dermal layer where keratinocytes were aliquoted, and systematized for 7 days.
5) After HuSkin additional culture for 2 days by simultaneously treating the inflammation inducing substances (poly I:C, 20µg/mL) and test substances (strain culture products selected in the present application, 0.5x concentration, 5%(v/v)) to T&R HuSkin obtained by culturing under ALI conditions for 7 days, changes in the skin improvement-related indexes were confirmed. (N=2 for each condition).
6) After obtaining IF fluorescence photographs, through a mean gray value of Image J, the fluorescence intensity value of each photograph (area of interest: total area of photograph) was quantified.

### Test result

### 1) Barrier improvement

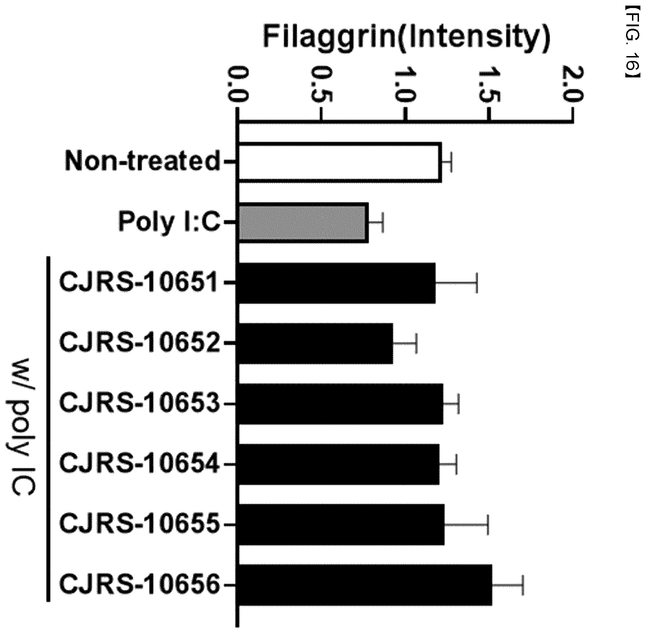

The expression level of Filaggrin, a barrier improvement index, was compared and analyzed in T&R HuSkin through IF staining, and the result was shown in FIG. 16.

As could be confirmed in FIG. 16, the expression level of Filaggrin increased equivalently or more compared to the negative control group (Poly I:C single treatment) in all the groups in which the culture products of the 6 strains selected in the present application (CJRS-10651 strain, CJRS-10652 strain, CJRS-10653 strain, CJRS-10654 strain, CJRS-10655 strain, or CJRS-10656 strain) were treated. It could be confirmed that the expression level of Filaggrin was recovered to the level equivalent to the normal control group (non-treated) in the group in which the CJRS-10651, CJRS-10653, CJRS-10654, or CJRS-10655 strain was treated among them, and it was confirmed that the expression level of Filaggrin further increased compared to the normal control group (non-treated) in the case of the group in which the culture products of the CJRS-10656 strain were treated.

### 2) Moisturizing alleviation

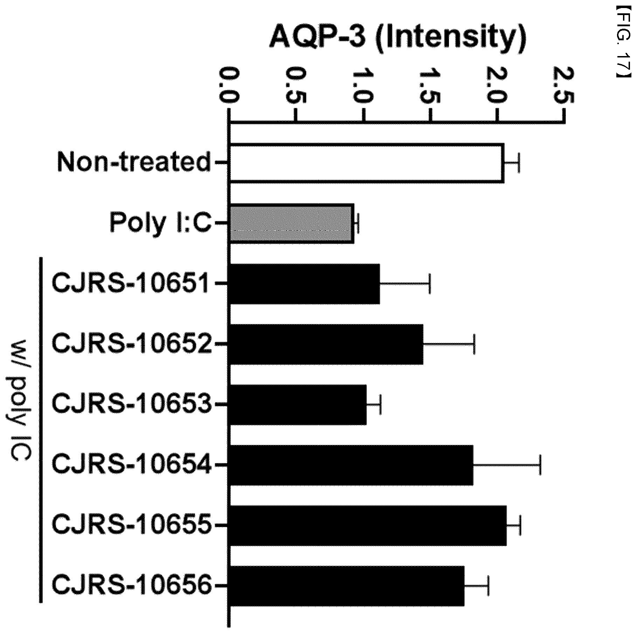

The expression level of Aquaporin-3, a moisturizing alleviation index, was compared and analyzed in T&R HuSkin through IF, and the result was shown in FIG. 17.

As could be confirmed in FIG. 17, in all the groups in which the culture products of the 6 strains selected in the present application (CJRS-10651 strain, CJRS-10652 strain, CJRS-10653 strain, CJRS-10654 strain, CJRS-10655 strain, or CJRS-10656 strain) were treated, the expression level of Aquaporin-3 increased equivalently or more compared to the negative control group (Poly I:C single treatment). It was confirmed that the expression level of Aquaporin-3 was recovered at an equivalent level compared to the normal control group (non-treated), in the groups in which CJRS-10654 strain, CJRS-10655 strain, or CJRS-10656 strain culture products were treated, among them.

### Example 12. Collagen index confirmation.

The degree of the toxicity of the culture products and collagen production and secretion by the culture products when the strain culture products selected in the present application were treated to human fibroblasts was confirmed.

### Experimental method

### 1) Human fibroblast culture

In the present experiment, human fibroblasts (human fibroblast cell, CCD-986sk cell) were used. Before one week prior to entering the present experiment, the human fibroblasts were cultured and maintained in FBS 10%(v/v) DMEM. Before 18 hours of the experiment, the human fibroblasts were seeded in a flat bottom 96 well plate at 1×10⁴/well.

### 2) Co-culture of strain culture products and human fibroblasts

After preparing using FBS 10%(v/v) DMEM so that the concentrations of the strain culture product undiluted solution ((in Experimental example 2, culture products in which strains were excluded by centrifuging the strain culture products (cell free supernatant)) and the strain culture products filtered to 3kDa or below prepared in Experimental example 2 were to be 10, 20, 30, 40 %(v/v), by adding 100 µL each to a plate comprising 100 µL of the human fibroblasts, they were treated at final concentrations of 5, 10, 15, 20 %(v/v) (total 200 µL/well) and cultured in a 37 °C incubator for a total of 24 hours.

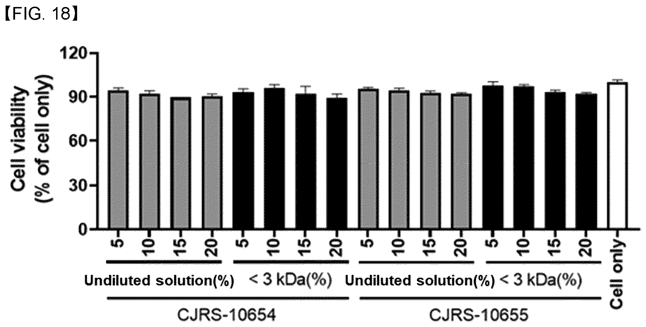

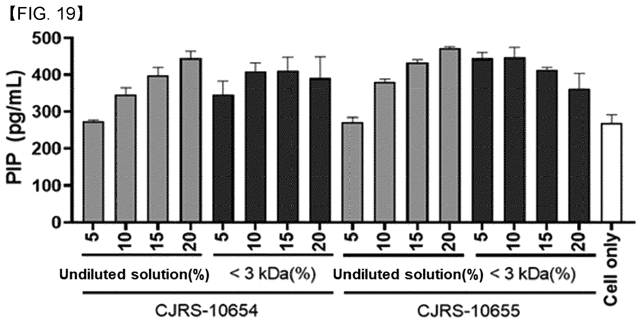

By confirming the effect on viability of the human fibroblasts and collagen production level of the culture products, the results were shown in FIG. 18 and FIG. 19. As a negative control group, a normal cell in which FBS 10%(v/v) DMEM was added (Cell only) was used.

### 3) Human fibroblast toxicity test

In order to confirm the viability level of the human fibroblasts remained after acquiring all the supernatant (culture supernatant) after 24 hours, after reacting in a 37°C incubator for 2 hours by treating cell counting kit 8 (Enzo, 5µL/well) and fresh RPMI media (95µL), the absorbance was measured at 450 nm, and the result was shown in FIG. 18.

As could be confirmed in FIG. 18, it was confirmed that there was no cytotoxicity in human fibroblasts at all the concentrations, when the strain culture product undiluted solution selected in the present application and the culture products filtered to 3kDa or less were treated.

### 4) Human fibroblast collagen producing function measurement

The collagen producing function was confirmed using an ELISA kit on procollagen type I C-peptide (PIP) secreted from human fibroblasts using the supernatant secured in the 3), and the result was shown in FIG. 19.

As could be confirmed in FIG. 19, it was confirmed that the PIP secretion increased equivalently or more compared to the control group (cell only) at all the concentrations, when the strain culture product undiluted solution selected in the present application and the culture products filtered to 3kDa or less were treated.

From the above description, those skilled in the art to which the present application belongs will be able to understand that the present application can be implemented in other specific forms without changing technical spirit or essential features thereof. In this regard, examples described above should be understood in all respects as illustrative and not restrictive. The scope of the present application should be interpreted as all changed or modified forms derived from the meaning and scope of claims described below than the above detailed description and equivalent concepts thereof are included in the scope of the present application.

### [ACCESSION NUMBER]

Name of Depository Authority : Korean Culture Center of Microorganisms
Accession number: KCCM13032P
Date of deposit: 20210813
Name of Depository Authority : Korean Culture Center of Microorganisms
Accession number: KCCM13033P
Date of deposit: 20210813
Name of Depository Authority : Korean Culture Center of Microorganisms
Accession number: KCCM13034P
Date of deposit: 20210813
Name of Depository Authority : Korean Culture Center of Microorganisms
Accession number: KCCM13035P
Date of deposit: 20210813
Name of Depository Authority : Korean Culture Center of Microorganisms
Accession number: KCCM13036P
Date of deposit: 20210813
Name of Depository Authority : Korean Culture Center of Microorganisms
Accession number: KCCM13037P
Date of deposit: 20210813

## Claims

1. A pharmaceutical composition for preventing or treating inflammatory skin disease, comprising a *Cutibacterium* sp. strain, a culture product of the strain, or a combination thereof,
wherein the *Cutibacterium* sp. strain comprises
(a) an Lrp/AsnC family transcriptional regulator or a gene encoding the same, and
(b) a lactococcin 972 family bacteriocin or a gene encoding the same.

2. The pharmaceutical composition according to claim 1, wherein the *Cutibacterium* sp. strain does not comprise at least one protein selected from the group consisting of the following (1) to (6) or a gene encoding the same:
(1) beta-glucuronidase or a gene encoding the same,
(2) 2-isopropylmalate synthase or a gene encoding the same,
(3) 3-isopropylmalate dehydratase or a gene encoding the same,
(4) type I-E CRISPR-associated protein Cse1/CasA or a gene encoding the same,
(5) type I-E CRISPR-associated protein Cas7/Cse4/CasC or a gene encoding the same, and
(6) CRISPR-associated helicase/endonuclease Cas3 or a gene encoding the same.

3. The pharmaceutical composition according to claim 1 or 2, wherein the inflammatory skin disease is at least one selected from the group consisting of hair loss, rosacea, erythema nodosum, erythema multiforme, keratosis pilaris, psoriasis, eczema, atopic dermatitis and acne.

4. An antimicrobial composition against inflammatory skin disease-causing bacterium, comprising a *Cutibacterium* sp. strain, a culture product of the strain, or a combination thereof,
wherein the *Cutibacterium* sp. strain comprises
(a) an Lrp/AsnC family transcriptional regulator or a gene encoding the same, and
(b) a lactococcin 972 family bacteriocin or a gene encoding the same.

5. The antimicrobial composition according to claim 4, wherein the *Cutibacterium* sp. strain does not comprise at least one protein selected from the group consisting of the following (1) to (6) or a gene encoding the same:
(1) beta-glucuronidase or a gene encoding the same,
(2) 2-isopropylmalate synthase or a gene encoding the same,
(3) 3-isopropylmalate dehydratase or a gene encoding the same,
(4) type I-E CRISPR-associated protein Cse1/CasA or a gene encoding the same,
(5) type I-E CRISPR-associated protein Cas7/Cse4/CasC or a gene encoding the same, and
(6) CRISPR-associated helicase/endonuclease Cas3 or a gene encoding the same.

6. The antimicrobial composition according to claim 4 or 5, wherein the inflammatory skin disease-causing bacterium is at least one selected from the group consisting of *Cutibacterium acnes* (*C. acnes*), *Cutibacterium avidum* (*C. avidum*), *Cutibacterium granulosum* (*C. granulosum*), *Staphylococcus aureus* (*S. aureus*), and *Staphylococcus epidermidis (S. epidermidis).*

7. A food composition for preventing or alleviating inflammatory skin disease, comprising a *Cutibacterium* sp. strain, a culture product of the strain, or a combination thereof,
wherein the *Cutibacterium* sp. strain comprises
(a) an Lrp/AsnC family transcriptional regulator or a gene encoding the same, and
(b) a lactococcin 972 family bacteriocin or a gene encoding the same.

8. The food composition according to claim 7, wherein the *Cutibacterium* sp. strain does not comprise at least one protein selected from the group consisting of the following (1) to (6) or a gene encoding the same:
(1) beta-glucuronidase or a gene encoding the same,
(2) 2-isopropylmalate synthase or a gene encoding the same,
(3) 3-isopropylmalate dehydratase or a gene encoding the same,
(4) type I-E CRISPR-associated protein Cse1/CasA or a gene encoding the same,
(5) type I-E CRISPR-associated protein Cas7/Cse4/CasC or a gene encoding the same, and
(6) CRISPR-associated helicase/endonuclease Cas3 or a gene encoding the same.

9. The food composition according to claim 7 or 8, wherein the inflammatory skin disease is at least one selected from the group consisting of hair loss, rosacea, erythema nodosum, erythema multiforme, keratosis pilaris, psoriasis, eczema, atopic dermatitis and acne.

10. A feed composition for preventing or alleviating inflammatory skin disease, comprising a *Cutibacterium* sp. strain, a culture product of the strain, or a combination thereof,
wherein the *Cutibacterium* sp. strain comprises
(a) an Lrp/AsnC family transcriptional regulator or a gene encoding the same, and
(b) a lactococcin 972 family bacteriocin or a gene encoding the same.

11. The feed composition according to claim 10, wherein the *Cutibacterium* sp. strain does not comprise at least one protein selected from the group consisting of the following (1) to (6) or a gene encoding the same:
(1) beta-glucuronidase or a gene encoding the same,
(2) 2-isopropylmalate synthase or a gene encoding the same,
(3) 3-isopropylmalate dehydratase or a gene encoding the same,
(4) type I-E CRISPR-associated protein Cse1/CasA or a gene encoding the same,
(5) type I-E CRISPR-associated protein Cas7/Cse4/CasC or a gene encoding the same, and
(6) CRISPR-associated helicase/endonuclease Cas3 or a gene encoding the same.

12. The feed composition according to claim 10 or 11, wherein the inflammatory skin disease is at least one selected from the group consisting of hair loss, rosacea, erythema nodosum, erythema multiforme, keratosis pilaris, psoriasis, eczema, atopic dermatitis and acne.

13. A *Cutibacterium* sp. strain comprising
(a) an Lrp/AsnC family transcriptional regulator or a gene encoding the same, and
(b) a lactococcin 972 family bacteriocin or a gene encoding the same.

14. The *Cutibacterium* sp. strain according to claim 13, wherein the *Cutibacterium* sp. strain does not comprise at least one protein selected from the group consisting of the following (1) to (6) or a gene encoding the same:
(1) beta-glucuronidase or a gene encoding the same,
(2) 2-isopropylmalate synthase or a gene encoding the same,
(3) 3-isopropylmalate dehydratase or a gene encoding the same,
(4) type I-E CRISPR-associated protein Cse1/CasA or a gene encoding the same,
(5) type I-E CRISPR-associated protein Cas7/Cse4/CasC or a gene encoding the same, and
(6) CRISPR-associated helicase/endonuclease Cas3 or a gene encoding the same.

15. The *Cutibacterium* sp. strain according to claim 13, wherein the *Cutibacterium* sp. Strain comprises a 16S rRNA gene of SEQ ID NO: 8.

16. The *Cutibacterium* sp. strain according to any one claims of 13 to 15, wherein the strain has at least one activity selected from the group consisting of anti-inflammatory activity, antibacterial activity against inflammatory skin disease-causing bacterum, skin regenerative activity, skin moisturizing activity, and preventing, alleviating and/or treating activity of inflammatory skin disease.
